(19) Europäisches Patentamt
European Patent Office
Office européen des brevets

(11) **EP 2 216 050 A1**

(12) **EUROPEAN PATENT APPLICATION**
published in accordance with Art. 153(4) EPC

(43) Date of publication:
**11.08.2010 Bulletin 2010/32**

(21) Application number: **08843543.3**

(22) Date of filing: **28.10.2008**

(51) Int Cl.:
***A61K 51/00*** *(2006.01)* ***C07D 471/04*** *(2006.01)*

(86) International application number:
**PCT/JP2008/069504**

(87) International publication number:
**WO 2009/057578 (07.05.2009 Gazette 2009/19)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **30.10.2007 JP 2007282364**

(71) Applicant: **Nihon Medi-Physics Co., Ltd.**
**Tokyo 136-0075 (JP)**

(72) Inventors:
- **TANIFUJI, Shigeyuki**
**Sodegaura-shi**
**Chiba 299-0266 (JP)**
- **NAKAMURA, Daisaku**
**Sodegaura-shi**
**Chiba 299-0266 (JP)**
- **TAKASAKI, Shinya**
**Sodegaura-shi**
**Chiba 299-0266 (JP)**
- **OKUMURA, Yuki**
**Sodegaura-shi**
**Chiba 299-0266 (JP)**

(74) Representative: **Wilhelms · Kilian & Partner**
**Patentanwälte**
**Eduard-Schmid-Straße 2**
**81541 München (DE)**

(54) **UTILIZATION OF NOVEL COMPOUNDS WITH AMYLOID AFFINITY AND METHOD OF PRODUCING THE SAME**

(57) The invention provides a reagent for detecting amyloid deposited in a biological tissue which can detect amyloid *in vitro* and *in vivo* with high sensitivity using a compound which has affinity with amyloid and is suppressed in toxicity such as mutagencity. The reagent for detecting amyloid deposited in a biological tissue comprises a compound represented by the following formula (1) or a salt thereof:

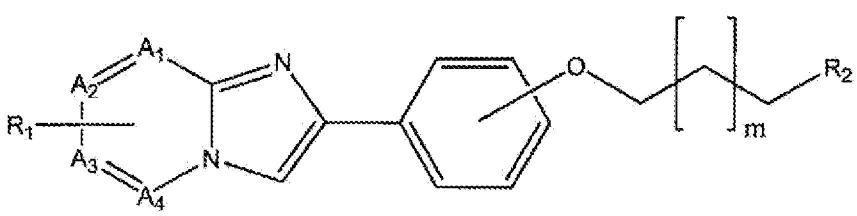

(1)

wherein $A^1$, $A^2$, $A^3$ and $A^4$ independently represent carbon or nitrogen; $R^1$ is a radioactive halogen substituent; $R^2$ is a group selected from the group consisting of hydrogen, hydroxyl group, methoxy group, carboxyl group, amino group, N-methylamino group, N,N-dimethylamino group and cyano group; and m is an integer of 0 to 2, provided that at least one of $A^1$, $A^2$, and $A^4$ represents a carbon, and $R^1$ binds to a carbon represented by $A^1$, $A^2$, $A^3$ and $A^4$.

EP 2 216 050 A1

## Description

### TECHNICAL FIELD

The present invention relates to use and process for production of a novel compound having affinity for amyloid, and especially relates to a reagent for detecting amyloid in biological tissues, which is useful for detection of amyloid at lesion sites in diagnosis of systemic amyloidosis diseases and other diseases with amyloid accumulation.

### BACKGORUND ART

Diseases with the onset of deposition of a fibrous protein called amyloid in various organs or tissues in bodies are generally referred to as amyloidosis. A feature common to amyloidosis is that the fibrous protein called amyloid which is enriched with the β-sheet structure is deposited at various organs systemically or at sites topically so that functional abnormalities are triggered in the organs or tissues. Amyloid generally refers to protein aggregates formed by aggregation of various amyloid precursor proteins such as amyloidβ, mutant transthyretin and β2-microglobulin in a living body. The amyloid has a characteristic structure enriched with β-sheet even if it is formed of any of the amyloid precursor proteins. Thus, compounds such as Congo-red and Thioflavin T capable of binding to β-sheet are **characteristic in that** they have affinity with amyloid.

Amyloidosis is classified into a systemic amyloidosis and a localized amyloidosis depending upon amyloid deposition patterns.

The systemic amyloidosis is a disease in which amyloid deposition occurs at various parts of the whole body. Examples of the systemic amyloidosis include familial amyloidosis in which amyloid produced in the liver is deposited in organs throughout the whole body so as to cause disorder, senile TTR amyloidosis in which amyloid is deposited in heart and a large joint such as hand joint, dialysis amyloidosis occurring at sites such as bones and joints of long-term dialysis patients, reactive AA amyloidosis (secondary amyloidosis) which is caused by deposition of amyloid derived from serum amyloid A which is an acute phase protein produced following a chronic inflammatory disease such as chronic rheumatism and immnunocytic amyloidosis in which amyloid derived from immunoglobulin is deposited in various organs throughout the whole body.

The localized amyloidosis is a disease in which amyloid deposition occurs only at some organs. Examples of the localized amyloidosis include brain amyloidosis such as Alzheimer's disease in which amyloid is deposited in brain, cerebrovascular amyloidosis and Creutzfeldt Jakob disease, endocrine amyloidosis in which amyloid is deposited in pancreatic inslet accompanied by type II diabetes and insulinoma or deposited in heart atrium, cutaneous amyloidosis in which amyloid is deposited in skin, and localized nodular amyloidosis in which nodular amyloid is deposited in skin and lungs.

Diagnosis of amyloidosis is, in case of the systemic amyloidosis, at first conducted by collecting a tissue from a region where biopsy is available such as skin, kidney and gastrointestinal tracts, and staining it with Congo-red of Thioflavin T. Congo-red is a fluorescent compound high in affinity with β-sheet structure of amyloid, and since Congo-red shows double refraction under polarization microscope due to its orientation, it can selectively stain amyloid deposition in tissues. Similarly, Thioflavin T is also a fluorescent compound having affinity with amyloid, and used similarly to Congo-red. After the tissue staining is found to be positive, definite diagnosis is conducted by means of immunostaining with an antibody or the like in combination therewith. However, positive diagnosis is often difficult by staining with Congo-red and Thioflavin T even under polarization microscope.

On the other hand, imaging diagnosis of the systemic amyloidosis has been considered with recent wide spread of image diagnosis devices such as PET, SPECT and MRI.

However, when Congo-red and Thioflavin T are labeled and used as probes for imaging diagnosis, they are problematic in that binding specificity to amyloid is inferior so that good detection sensitivity cannot be obtained.

Further, since Congo-red has carcinogenicity, it cannot be used for diagnosis of human body.

Thus, bis-(3-hydroxycarbonyl-4-hydroxy)styrylbenzene (BSB) as a Congo-red derivative and derivatives thereof have been proposed as a fluorescent reagent for detecting amyloid which is high in affinity and detection sensitivity for systemic amyloid and can be used for in vivo detection (non-Patent Document 14, Patent Document 7) . It has been reported that BSB is high in affinity for amyloid caused by brain amyloidosis and systemic amyloidosis, and has no bensizine structure in its structure, and thus it has little carcinogenic problem and can be radioactive-labeled for use as a probe for imaging diagnosis.

On the other hand, for Alzheimer's disease (hereinafter, referred to as AD) which is a typical brain amyloidosis, attempts have already been made to detect AD in vivo using a compound having high affinity with amyloid as a marker since it is impossible to collect a biopsy.

Many of such probes for imaging diagnoses of cerebral amyloid are hydrophobic low-molecular weight compounds that are high in affinity with amyloid and high in cerebral transferabillty and are labeled with various radioactive species such as $^{11}C$, $^{18}F$ and $^{123}I$. For example, reports tell $^{11}C$ or radioactive halogen labeled forms of compounds including various

thioflavin derivatives such as 6-iodo-2-[4-(N,N-dimethylamino)phenyl]benzothiasole (hereinafter referred to as TZDM) and 6-hydroxy-2-[4-(N-methylamino)phenyl]benzothlazole (hereinafter referred to as 6-OH-BTA-1) (Patent Document 1, Non-Patent Document 3); stilbene compounds such as (E)-4-methylamino-4 - hydroxystilbene (hereinafter referred to as SB-13) and (E)-4-dimethylamino-4-iodostilbene (hereinafter referred to as m-I-SB) (Patent Document 2, Non-Patent Document 4, Non-Patent Document 5); benzoxazole derivatives such as 6-iodo-2-[4-(N,N-dimethylamino)phenyl] benzoxazole (hereinafter referred to as IBOX) and 6-[2-(fluoro)ethoxy]-2-[2-(2-dimethylaminothlazol-5-yl)ethenyl]benzoxazole (Non-Patent Document 6, Non-Patent Document 7), DDNP derivatives such as 2-(1-{6-[(2-fluoroethyl) (methyl) amino]-2-naphthyl}ethylidene)malononltrile (hereinafter referred to as FDDNP) (Patent Document 4, Non-Patent Document 8); and imidaxopyridine derivatives such as 6-iodo-2-[4-(N,N-dimethylamino)phenyl]imidazo[1,2-a]pyridine (hereinafter referred to as IMPY) (Patent Document 3, Non-Patent Document 9). Further, some of these probes for imaging diagnosis have been studied on human imaging and have been reported to show a significant radioactivity accumulation in AD patient's brain compared with normal persons (Non-Patent Document 10, Non-Patent Document 11, Non-Patent Document 12, and Non-Patent Document 13).

International Publication No. WO2007/002540 pamphlet discloses a series of compounds with a group having affinity with amyloid, to which a radioisotope labeling site is attached via ethylene glycol or polyethylene glycol (Patent Document 5).

International Publication No. WO2007/063946 pamphlet discloses a series of compounds to which a five-membered aromatic heterocyclic group is attached in order to prevent them from being metabolized in brain (Patent Document 6).

[Patent Document 1] JP-T-2004-506723

[Patent Document 2] JP-T-2005-504055

[Patent Document 3] JP-T-2005-512945

[Patent Document 4] JP-T-2002-523383

[Patent Document 5] International Publication No. WO2007/002540 pamphlet

[Patent Document 6] International Publication No. WO2007/063946 pamphlet

[Patent Document 7] International Publication No. WO2005/016888 pamphlet

[Non-Patent Document 1] J. A. Hardy & G. A. Higgins, "Alzheimer's Disease: The Amyloid Cascade Hypothesis.", Science, 1992, 256, p.184-185

[Non-Patent Document 2] G. McKhann et al., "Clinical diagnosis of Alzheimer's disease: Report of the NINCDS-ADRDA Work Group under the auspices of Department of Health and Human Services Task Force on Alzheimer's Disease.", Neurology, 1984, 34, p.939-944

[Non-Patent Document 3] Z.-P. Zhuang et al., "Radioiodinated Styrylbenzenes and Thioflavins as Probes for Amyloid Aggregates.", J. Med. Chem., 2001, 44, p.1905-1914

[Non-Patent Document 4] Masahiro Ono et al., "11C-labeled stilbene derivatives as Aβ-aggregate-specific PET imaging agents for Alzheimer's disease.", Nuclear Medicine and Biology, 2003, 30, p.565-571

[Non-Patent Document 5] H. F. Kung et al., "Novel Stilbenes as Probes for amyloid plaques.", J. American Chemical Society, 2001, 123, p.12740-12741

[Non-Patent Document 6] Zhi-Ping Zhuang et al., "IBOX(2-(4-dimethylaminophenyl)-6-iodobensoxazole): a ligand for imaging amyloid plaques in the brain.", Nuclear Medicine and Biology, 2001, 28, p.887-894

[Non-Patent Document 7] Furumoto Y et al., "[11C]BF-227: A New 11C-Labeled 2-Ethenylbenzoxazole Derivative for Amyloid-β Plaques Imaging.", European Journal of Nuclear Medicine and Molecular Imaging, 2005, 32, Sup.1, P759 [Non-Patent Document 8] Eric D. Agdeppa et al., "2-Dialkylamino-6-Acyimalononitrile Substituted Naphthalenes (DDNP Analogs) : Novel Diagnostic and Therapeutic Tools in Alzheimer's Disease.", Molecular Imaging and Biology, 2003, 5, p.404-417

[Non-Patent Document 9] Zhi-Ping Zhuang et al., "Structure-Activity Relationship of Imidazo[1,2-a]pyridines as Ligands for Detecting β-Amyloid Plaques in the Brain.", J. Med. Chem, 2003, 46, p.237-243

[Non-Patent Document 10] W. E. Klunk et al., "Imaging brain amyloid in Alzheimer's disease with Pittsburgh Compound-B.", Ann. Neurol., 2004, 55, p.306-319

[Non-Patent Document 11] Nicolaas P. L. G. Verhoeff et al., "In-Vivo Imaging of Alzheimer Disease β-Amyloid With [11C] SB-13 PET.", American Journal of Geriatric Psychiatry, 2004, 12, p.584-595

[Non-Patent Document 12] Hiroyuki Arai et al., "[11C]-BF-227 AND PET to Visualize Amyloid in Alzheimer's Disease Patients", Alzheimer's & Dementia: The Journal of the Alzheimer's Association, 2006, 2, Sup. 1, S312

[Non-Patent Document 13] Christopher M. Clark et al., "Imaging Amyloid with I123 IMPY SPECT", Alzheimer's & Dementia: The Journal of the Alzheimer's Association, 2006, 2, Sup. 1, S342

[Non-Patent Document 14] D. M. Skovronsky et al., Proc. Natl. Acad. Sci., 2000, 97, 7609

DISCLOSURE OF THE INVENTION

PROBLEMS TO BE SOLVED BY THE INVENTION

As described above, various compounds are disclosed as probes for imaging diagnosis for amyloid, and researched for clinical application.
Experiments in normal mice show that [$^{125}$I]-labeled TZDM, IBOX and m-I-SB are all transferred into brain 2 minutes after administration. However, these compounds are insufficient in clearance from normal tissues, and tend to accumulate gradually in brain as time passes after administration (JP-T-2005-512945; Zhi-Ping Zhuang et al., Nuclear Medicine and Biology, 2001, 28, p.887-894; H. F. Kung et al., J. Am. Chem. Soc., 2001, 123, p.12740-12741). When the clearance from normal tissues is insufficient, a problem arises in that sufficient contrast cannot be obtained at amyloid accumulation sites. [$^{11}$C]-labeled SB-13 shows a clearance from normal tissues in experiments in rats, however, it cannot be said that the clearance is sufficiently fast (Masahiro One et al., Nuclear Medicine and Biology, 2003, 30, p.565-571).
Meanwhile, it is revealed that compounds having an imidazopyridine skeleton such as IMPY have a property of transferring to brain and accumulating at amyloid after administration, and also have an excellent property of rapid clearance from normal tissues unlike the above-described compounds, as a result of experiments using [$^{125}$I]-labeled compounds. However, IMPY is a compound positive in reverse mutation test. In order to use this compound as a probe for imaging diagnosis, sufficient care must be taken about dosage and administration manner (International Publication No. WO03/106439 pamphlet).
FDDNP is also reported to be positive in reverse mutation test (International Publication No. WO03/106439 pamphlet).
A preferable probe targeting amyloid for imaging diagnosis would be a compound that is excellent in affinity with amyloid and sufficiently rapid in clearance from normal tissues like IMPY but is suppressed in toxicity such as mutagenicity. However, no compound with such properties has been disclosed.
Furthermore, in accordance with results of our studies, it has been confirmed that IMPY accumulates unspecifically on white matter or other sites where amyloid is not deposited. As an AD diagnostic agent, a compound must be used which is suppressed in unspecific accumulation on sites other than amyloid deposition, but such as compound has not been disclosed.
The present invention has been made under such circumstances where various compounds as probes targeting amyloid have been disclosed but there has been no compound which is confirmed to have a clinically tolerable property, and aims at making it possible to detect amyloid in vivo or *in vitro* with high sensitivity by providing a novel compound having affinity with amyloid.

MEANS FOR SOLVING THE PROBLEM

The present inventors have found that a specific novel compound with an imidazopyridine-phenyl skeleton or a skeleton similar thereto whose phenyl group has a carbon to which an oxygen atom is attached has affinity with amyloid and is low in toxicity such as mutagenicity, and amyloid can be detected in vivo or in vitro with high sensitivity by use of a series of the compound as a probe. Thus, the present invention has been completed.
That is, according to one aspect of the present invention, a reagent for detecting amyloid deposited on a biological tissue is provided, which comprises a compound represented by the following formula (1):
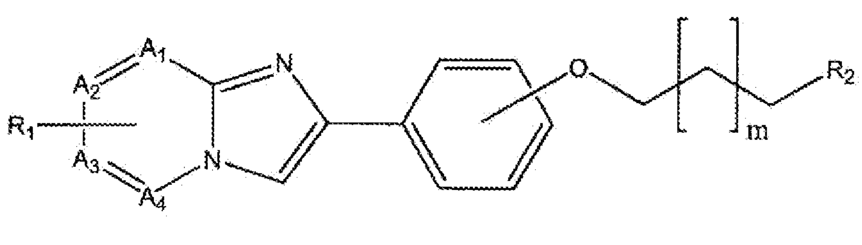

(1)

or a salt thereof. Specifically, a compound represented by the above formula (1) or a salt thereof provides a highly-specific reagent for detecting amyloid. A highly-specific reagent for detecting amyloid here refers to a reagent which has a property of accumulating at amyloid and hardly accumulating at other sites or rabidly clearing the sites even if it accumulates there, and thus shows high specificity to amyloid imaging in a certain period of time after administration.
The biological tissue can be various tissues at which amyloid is known to deposit in amloidosis.
Typical examples of such biological tissues include brain, heart, lung, pancreas, bone and joint, and as the most typical

biological tissue, mention may be made of brain. The typical amyloidosis in case of targeting a brain includes Alzheimer's disease and dementia with Lewy bodies.

In the formula (1), $A_1$, $A_2$, $A_3$ and $A_4$ independently represent a carbon or nitrogen, and it is necessary that at least one of these represents a carbon. Preferably, 3 or more of $A_1$, $A_2$, $A_3$ and $A_4$ represent carbons, and more preferably, all of them represent carbons. In the formula (1), $R^1$ binds to a carbon represented by $A_1$, $A_2$, $A_3$ or $A_4$. A binding site for $R^1$ is preferably a carbon represented by $A_3$, that is, a carbon at 6-position.

In the formula (1), $R^1$ is a radioactive halogen substituent. As $R^1$, can be used various radioactive halogens, preferably a radioactive halogen selected from the group consisting of $^{18}F$, $^{75}Br$, $^{76}Br$, $^{123}I$, $^{124}I$, $^{125}I$ and $^{131}I$, and more preferably $^{18}F$ or $^{123}I$.

$R^2$ is a group selected from the group consisting of a hydrogen, hydroxyl group, methoxy group, carboxyl group, amino group, N-methylamino group, N, N-dimethylamino group and cyano group. $R^2$ is preferably a hydrogen, hydroxyl group, carboxyl group or amino group, more preferably hydrogen or hydroxyl group and particularly preferably hydroxyl group.

In addition, m is an integer of 0 to 2.

According to a particularly preferable embodiment, a compound of the formula (1) of the present invention is selected from the group consisting of 2-(4 - ethoxyphenyl)-6-[$^{123}I$]iodoimidazo[1,2-a]pyridine, 2-(4 - ethoxyphenyl)-6-[$^{125}I$]iodoimidazo[1,2-a]pyridine, 2-(4 - ethoxyphenyl)-6-[$^{131}I$]iodoimidazo[1,2-a]pyridine, 2-[4 - (2 -hydrozye-thoxy)phenyl]-6-[$^{123}I$]iodoimidazo[1,2-a]pyridine, 2-[4-(2-hydroxyethoxy)phenyl]-6-[$^{125}I$]iodoimidazo[1,2-a]pyridine, 2-[4-(2-hydroxyethoxy)phenyl]-6-[$^{131}I$]iodoimidazo[1,2-a]pyridine, 2-[4-(3-hydroxypropoxy)phenyl]-6-[$^{123}I$]iodoimidazo[1,2-a]pyridine, 2-[4-(3-hydroxypropoxy)phenyl]-6-[$^{125}I$]iodoimidazo[1,2-a]pyridine, 2-[4-(3-hydroxypropoxy)phenyl]-6-[$^{131}I$]iodoimidazo[1,2-a]pyridine, 2-(3-ethoxyphenyl)-6-[$^{123}I$]iodoimidazo[1,2-a]pyridine, 2-(3 -ethoxyphenyl)-6-[$^{125}I$]iodoimidazo[1,2-a]pyridine, 2-(3 -ethoxyphenyl)-6-[$^{131}I$]iodoimidazo[1,2-a]pyridine, 2-[3 -(2 -hydroxyethoxy)phenyl]-6-[$^{123}I$]iodoimidazo[1,2-a]pyridine, 2-[3-(2-hydroxyethoxy)phenyl]-6-[$^{125}I$]iodoimidazo[1,2-a]pyridine, 2-[3-(2-hydroxyethoxy)phenyl]-6-[$^{131}I$]iodoimidazo[1,2-a]pyridine, 2-[3-(3-hydroxypropoxy)phenyl]-6-[$^{123}I$]iodoimidazo[1,2-a]pyridine, 2-[3-(3-hydroxypropoxy)phenyl]-6-[$^{125}I$]iodoimidazo[1,2-a]pyridine and 2-[3-(3-hydroxypropoxy)phenyl]-6-[$^{131}I$]iodoimidazo[1,2-a]pyridine, and a preferable reagent for detecting amyloid of the present invention is a reagent for detecting amyloid comprising these compounds or a salt thereof.

The above compound of the formula (1) is a novel compound, and according to another aspect of the present invention, a process for production of a radioactive halogen-labeled organic compound is provided, which comprises a step of preparing a reaction solution containing, together with a radioactive halogen ion, a compound represented by the following formula (2):

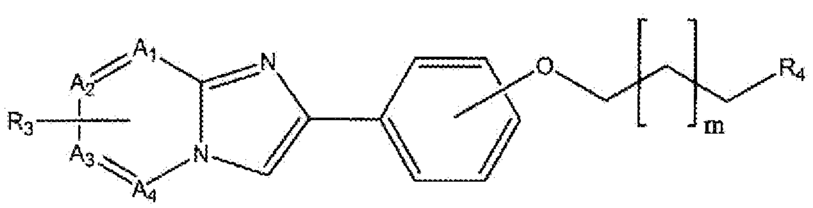

(2)

wherein $A_1$, $A_2$, $A_3$ and $A_4$ independently represent a carbon or nitrogen, $R^3$ is a group selected from the group consisting of a non-radioactive halogen substituent, nitro group, trialkylammonium group having alkyl chains with 1 to 4 carbon atoms, trialkylstannyl substituent having alkyl chains with 1 to 4 carbon atoms and triphenylstannyl group,
$R^4$ is a group selected from the group consisting of a hydrogen, hydroxyl group, methoxy group, carboxyl group, amino group, N-methylamino group, N,N-dimethylamino group and cyano group, and
m is an integer of 0 to 2,
provided that at least one of $A_1$, $A_2$, $A_3$ and $A_4$ represents a carbon, and $R^3$ binds to a carbon represented, by $A_1$, $A_2$, $A_3$ or $A_4$,
or a salt thereof,
and a step of giving a reaction condition to the reaction solution to synthesize a compound represented by the following formula (1):

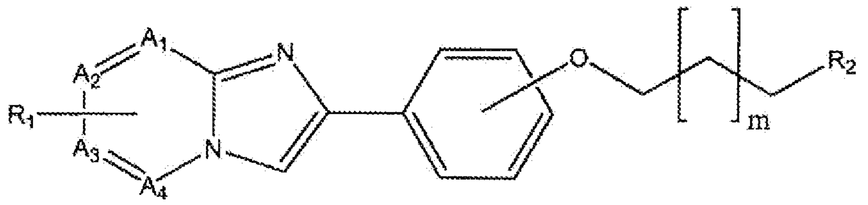

(1)

wherein $A_1$, $A_2$, $A_3$ and $A_4$ independently represent a carbon or nitrogen,
$R^1$ is a radioactive halogen substituent,
$R^5$ is a group selected from the group consisting of a hydrogen, hydroxyl group, methoxy group, carboxyl group, amino group, N-methylamino group, N,N-dimethylamino group and cyano group, and
m is an integer of 0 to 2,
provided that at least one of $A_1$, $A_2$, $A_3$ and $A_4$ represents a carbon, and $R^1$ binds to a carbon represented by $A_1$, $A_2$, $A_3$ or $A_4$, or a salt thereof.

In the formula (2), $A^1$, $A^2$, $A^3$ and $A^4$ independently represent a carbon or nitrogen, and it is necessary that at least one of these represents a carbon. Preferably, 3 or more of $A^1$, $A^2$, $A^3$ and $A^4$ represent carbons, and more preferably, all of them represent carbons. In the formula (2), $R^3$ binds to a carbon represented by $A^1$, $A^2$, $A^3$ or $A^4$. A binding site for $R^3$ is preferably a carbon represented by $A^3$, that is, a carbon art 6-position.

The step of preparing a reaction solution comprising a precursor compound shown above in the formula (2) or a salt thereof and a radioactive halogen ion can be conducted, for example, by dissolving the precursor compound or a salt thereof in an inert organic solvent, and adding thereto a radioactive halogen ion-containing solution which has been obtained with a known method.
As the inert organic solvent, various solvents which do not have reactivity with the precursor compound or a salt thereof and a radioactive halogen ion can be used, for example, when the radioactive halogen ion to be used is a radioactive iodine, methanol can preferably be used, and when a radioactive halogen ion to be used is a radioactive fluorine, acetonitrile can preferably be used.
The reaction condition to be given for the reaction solution in the step of synthesizing a compound represented above in the formula (1) or a salt thereof is not particularly limited as long as it is a condition in which a reaction of substituting $R^3$ of a compound of the formula (2) with the radioactive halogen ion added to the reaction solution is allowed to proceed, and a known reaction condition that fits kinds of the radioactive halogen ion can be used.

In the process for producing the radioactive halogen-labeled organic compound of the present invention, as the radioactive halogen ion, for example, $^{18}F$, $^{75}Br$, $^{76}Br$, $^{123}I$, $^{124}I$, $^{125}I$ or $^{131}I$ can be used.
When a compound of the formula (1) in which a halogen substituent represented by $R^1$ is $^{123}I$, $^{124}I$, $^{125}I$ or $^{131}I$ is produced, $^{123}I$ ion, $^{124}I$ ion, $^{125}I$ ion or $^{131}I$ ion is respectively used as a radioactive halogen ion, and as a compound of the formula (2), preferably used is a compound in which $R^3$ is a iodine, bromine, trialkylstannyl substituent having alkyl gumps with from 1 to 4 carbon atoms or triphenylstannyl substituent, and more preferably a compound in which $R^3$ is iodine, trimethylstannyl substituent, tributylstannyl substituent and triphenyl substituent.
When a compound of the formula (1) in which a radioactive halogen substituent represented by $R^1$ is $F^{18}$ is produced, $F^{18}$ ion is used as a radioactive halogen ion and as a compound of the formula (2), preferably used is a compound in which $R^3$ is a nitro substituent or trialkylammonium substituent having alkyl groups with from 1 to 4 carbon atoms.
When a compound of the formula (1) in which a halogen substituent represented by $R^1$ is $^{75}Br$ or $^{76}Br$ is produced, $^{75}Br$ ion or $^{76}Br$ ion is respectively used as a radioactive halogen ion and as a compound of the formula (2), preferably used is a compound in which $R^3$ is a bromine.

According to still another aspect of the present invention, a precursor compound for synthesizing a radioactive halogen-labeled organic compound is provided, which is represented by the following formula (2):

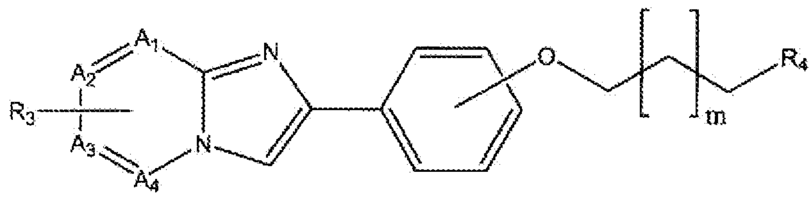

(2)

or a salt thereof.

In the formula (2), $A_1$, $A_2$, $A_3$ and $A_4$ independently represent a carbon or nitrogen, and it is necessary that at least one of these represents a carbon. Preferably, 3 or more of $A_1$, $A_2$, $A_3$ and $A_4$ represent carbons, and more preferably, all of them represent carbons. In the formula (2), $R^3$ binds to a carbon represented by $A_1$, $A_2$, $A_2$ or $A_4$. A binding site for $R^3$ is preferably a carbon represented by $A_3$, that is, a carbon at 6-posltion.

In the formula (2), $R^3$ is a group selected from the group consisting of a non-radioactive halogen substituent, nitro group, trialkylammonium group having alkyl chains with 1 to 4 carbon atoms, trialkylstannyl substituent having alkyl chains with 1 to 4 carbon atoms and triphenylstannyl group. As a non-radioactive halogen substituent, a halogen capable of being a target of nucleophilic substitution reactions using a radioactive fluorine or a halogen capable of being a target of isotope exchange reactions with a radioactive iodine can be used, and preferably chlorine, iodine or bromine can be used. As a trialkylstannyl substituent, various substituents can be used, and trimethylstannyl substituent and tributylstannyl substituent are preferably used.

$R^4$ is a group selected from the group consisting of a hydrogen, hydroxyl group, methoxy group, carboxyl group, amino group, N-methylamino group, N.N-dimethylamino group and cyano group. $R^4$ is preferably a hydrogen, hydroxyl group, carboxyl group or amino group, more preferably hydrogen or hydroxyl group and particularly preferably hydroxyl group.

In addition, in is an integer of 0 to 2.

## EFFECT OF THE INVENTION

In accordance the present invention, a reagent for detecting amyloid, which has affinity with amyloid and is suppressed in toxicity such as mutagenicity and thus can be used for *in vitro* and *in vivo* detection of amyloid related to a wide range of amyloidosis, can be obtained as well as a process for production thereof and a production intermediate thereof.

## BEST MODE FOR CARRYING OUT THE INVENTION

(A method for synthesis of a precursor compound for preparation of a radioactive halogen-labeled organic compound)

Hereinafter, a method for synthesis of a precursor compound for preparation of a radioactive halogen-labeled organic compound according to an embodiment of the present invention will be described, taking the case of 6-tributylstannyl-(2-hydroxyethoxy)phenyl]imidazo[1,2-a]pyridine as an example.

For the synthesis of 6-tributylstannyl-2-[4-(2-hydroxyethoxy)phenyl]imidazo[1,2-a]pyridine, first, 4-hydroxyacetophenone is allowed to react with cupric bromide to prepare 2-bromo-4 -hydroxyacetophenone (Fig. 1, Step 1). In this instance, the reaction can be conducted in accordance with ordinary methods, for example, the method described in a literature, King, L. Carroll & Ostrum, G. Kenneth, Journal of Organic Chemistry, 1964, 29(12), p.3459-3461.

Then, 2-bromo-4 -hydroxyacetophenone as prepared above is allowed to react with 2-amino-5-iodopyridine to prepare 2-(4 -hydroxyphenyl)-6-iodoimidazo[1,2-a]pyridine (Fig. 1, Step 2). This step can be done according to the following procedure.

First, 2-bromo-4 -hydroxyacetophenone and 2-amino-5-iodopyridine are dissolved in an inactive solvent such as acetonitrile, and are allowed to react with each other at a reflux temperature for 2 to 6 hours to produce 2-(4 -hydroxyphenyl)-6-iodoimidazo[1,2-a]pyridine hydrobromide salt as white precipitates. The solvent used in this instance may be acetonitrile or another solvent that is usually employed in a similar reaction, for example, any acetone. The reaction temperature may be a temperature allowing refluxing, for example, 110°C when the solvent is acetonitrile. The amount of the solvent to be used may be an amount sufficient to effect the reaction, however, it should, be noted that if the solvent is too much, it will become difficult to obtain precipitates of reaction products. For example, when 2-bromo-4 -hydroxyacetophenone in an amount corresponding to 10 mmol is used for the reaction, the amount of a solvent to be used can be about 40 to 80 mL.

Next, the reaction solution is filtered to recover the precipitates. The white precipitates are suspended in a mixed solution of methanol/water (1:1). Then, an aqueous saturated solution of sodium, hydrogencarbonate is added thereto in a very excessive amount relative to the suspended precipitates to release 2-(4 -hydroxyphenyl)-6-iodoimidazo [1,2a]pyridine as precipitates. The newly generated precipitates are filtered to recover 2-(4-hydroxyphenyl)-6-iodoimidazo [1,2-a]pyridine as the target compound in this step (Fig. 1, Step 2). The amount of the mixed solution of methanol/water is not specifically limited as long as it is sufficient to effect the reaction. However, it should be noted that if the amount of the mixed solution is too much, precipitation of products will be hindered. For example, when 2-bromo-4-hydroxyacetophenone in an amount corresponding to 10 mmol is used, the mixed solution of methanol/water may be used in an amount of about 40 to 100 mL. The amount of sodium hydrogencarbonate is not specifically limited as long as it is very excessive relative to the above-described precipitates as reaction substrates. For example, when the reaction is effected under the above-described conditions, the amount of an aqueous saturated solution of sodium hydrogencarbonate to be added to the reaction solution can be about 50 mL.

Here, 2-bromoethanol and t-butyldiphenylchlorosilane (TBDPSC1) are reacted with each other to prepare 1-bromo-2-(t-butyldiphenylsiloxy)ethane (Fig. 1, Step 3), separately. In this instance, the reaction can be carried out in accordance with ordinary methods, for example, the method described in a literature (Organic Syntheses, Coll. Vol. 10, p.170 (2004); Vol. 79, p.59 (2002)).

Then, the 2-(4'-hydroxyphenyl)-6-iodoimidazo[1,2-a]pyridine prepared above is sufficiently dried, dissolved in N,N-dimethylformamide, and potassium carbonate and 1-bromo-2-(t-butyldiphenylsiloxy)ethane were added thereto. After this mixture was stirred at about 90°C for about 2 hours, a saturated sodium chloride solution is added followed by extraction with ethyl acetate, and the ethyl acetate layer is concentrated and subjected to chromatogram purification to obtain 2-[4-(2-t-butyldiphenylsiloxyethoxy)phenyl]-6-iodoimidazo[1,2-a]pyridine (Fig. 1, Step 4). The amount of potassium carbonate may be an amount that can neutralize hydrobromic acid generated from 1-bromo-2-(t-butyldiphenylsiloxy)ethane during the reaction, and is typically about double triple the other reactant 1-bromo-2-(t-butyldiphenylsiloxy) ethane in molar ratio. Further, the 1-bromo-2-(t-butyldiphenylsiloxy)ethane can be used in an excessive amount relative to the reaction substrate, and is typically about 1.5 times the reaction substrate 2-(4'-hydroxyphenyl)-6-iodoimidazo[1,2-a]pyridine in molar ratio.

Then, t-butyldiphenylsilyl group of the obtained 2-[4-(2-t-butyldiphenylsiloxyethoxy)phenyl]-6-iodoimidazo[1,2-a]pyridine is deprotected using tetrabutylammonium fluoride to obtain 2-[4-(2-hydroxyethoxy)phenyl]-6-iodoimidaso[1,2-a]pyridine (Fig. 1, Step 5). In this instance, the reaction can be carried out in accordance with ordinary methods, for example, the method described in a literature (Organic Syntheses, Coll. Vol. 9, p.417 (1998); Vol. 74, p.248 (1997)).

The obtained 2-[4-(2-hydroxyethoxy)]phenyl]-6-iodoimldazo[1,2-a]pyridine is dissolved in dioxane, and triethylamine is added to the solution, followed by addition of bis (tributyltin) and a catalytic amount of tetrakis-triphenylphosphine palladium. This reaction solution is heated at about 90°C to effect reaction for about. 24 hours, and then a solvent is distilled off and chromatographic purification is performed to obtain 6-tributylstannyl-2-[4-(2-hydroxyethoxy)]phenyl]imidazo[1,2-a]pyridine as the target compound (Fig. 2, Step 1). The amount of bis(tributyltin) to be used in this instance may be an amount satisfying a condition where it is excessive relative to the reaction substrate, specifically, it is about 1.5 times in molar ratio relative to the reaction substrate 2-[4-(2-hydroxyethoxy)phenyl]-6-iodoimidazo[1,2-a]pyridine.

When a compound with a substituent at the 6-position in the imidazo pyridine ring being a trialkylstannyl substituent other than the tributylstannyl substituent is obtained, various bis(trialkyltin)s that fit purposes can be used instead of bis(tributyltin) in Fig. 2, Step 1. For example, when a compound having a trimethylstannyl substituent as a substituent at the 6-position is synthesized, a reaction similar to the above can be performed using bis(trimethyltin) in Fig. 2, Step 1.

A compound with an imidazopyridine ring in which the binding site for the functional group is a carbon atom other than the carbon at 6-position can be obtained by using a compound with a pyridine ring to which iodine is bonded at a site instead of 2-amino-5-iodopyridine in Fig. 1, Step 2. For example, when a binding site for the functional group is the carbon at 8-position in the imidazopyridine ring, 2-amino-3-iodopyridine may be used instead of 2-amino-5-iodopyridine in Fig. 1, Step 2.

(A method for synthesis of a radioactive halogen-labeled organic compound)

Hereinafter, a method for production of a radioactive halogen-labeled organic compound according to another aspect of the present invention will be described, taking the case of radioactive iodine-labeled compounds as an example.

The synthesis of radioactive iodine-labeled compounds can be performed by dissolving the labeling precursor compound prepared as above procedure in an inert organic solvent, adding thereto a [$^{123}$I] sodium iodide solution or the like obtained by known methods, and adding thereto an acid and an oxidising agent. As an inert organic solvent dissolving the labeling precursor compound, various solvents having no reactivity with the labeling precursor, [$^{123}$I] sodium iodide and the like can be used, and preferably methanol can be used.

As the acid, may be used various ones, and preferably hydrochloric acid.

The oxidizing agent is not particularly limited as long as it can effect the oxidation of iodine in the reaction solution, and is preferably hydrogen peroxide or peracetic acid. The amount of the oxidizing agent to be added may be an amount sufficient to oxidize iodine in the reaction solution.

A compound labeled with a radioactive halogen other than iodine can be synthesized by labeling a labeling precursor that fits a purpose of synthesis with a radioactive halogen that fits the purpose. For example, in order to synthesize 6-[$^{18}$F]fluoro-2-[4-(2-hydroxyethoxy)phenyl]imidazo[1,2-a]pyridine, the labeling precursor 2-[4-(2-hydroxyethoxy)phenyl]-6-nitroimidazo[1,2-a]pyridine can be reacted with [$^{18}$F]fluoride ion in the presence of a phase transfer catalyst and potassium carbonate.

(Methods for preparing and using a detection reagent in accordance with the present invention)

Amyloid has many different structures depending upon kinds of precursor protein, but they are common in a point of having βsheet structure. It has been known that many staining reagents for amyloid such as Thioflavin T and Congo-red target the β-sheet structure, and has the staining ability equally to different kinds of amyloid.

The compound of the formula (1) according to the present invention has affinity with amyloid originated from amyloid β-protein (hereinafter referred to as Aβ) as a precursor compound, and also has an activity of inhibiting the binding to amyloid of Thioflavin T which is known to bind to a wide range of amyloid.

Therefore, the compound of the formula (1) according to the present invention is considered, to have affinity with βsheet structure of amyloid protein like Thioflavin T. This suggests that the compound of the formula (1) according to the present invention has the same affinity with various amyloids.

That is, the reagent for detecting amyloid according to the present invention can be used for diagnosing the systemic amyloidosis and localized amyloidosis similarly to Thioflavin T and Congo-red. The systemic amyloidosis includes immunoglobulin amyloidosis, reactive AA amyloidosis, familial amyloidosis, dialysis amyloidosis and senile amyloidosis. The localized amyloidosis includes brain amyloidosis, endocrine amyloidosis, cutaneous amyloidosis and localized nodular amyloidosis.

The reagent for detecting amyloid according to the present invention can be not only used as a reagent used *in vitro* for biopsy, but also used as a reagent used *in vivo* as a radioactive diagnostic agent.

The reagent for detecting amyloid according to the present invention can be prepared as a solution which comprises the radioactive halogen-labeled compound of the above formula (1) blended in water, a physiological saline solution or a Ringer's solution optionally adjusted to an appropriate pH, like other commonly-known radioactive diagnostic agents. In this instance, concentration of the present compound should be adjusted to not more than the concentration at which stability of the present compound is ensured. Dosage of the present compound is not specifically limited, as long as it is sufficient to obtain an image of distribution of an administered agent. For example, in case of iodine-123($^{123}$I)-labeled compounds and fluorine-18($^{18}$F)-labeled compounds, about 50 to 600 MBq per adult body of 60 kg weight can be administered intravenously or locally. Distribution of administered agents can be imaged by known methods. For example, iodine-123($^{123}$I)-labeled compounds can be imaged by a SPEC apparatus while fluorine-18($^{18}$F)-labeled compounds can be imaged by a PET apparatus.

By administering the reagent for detecting amyloid according to the present invention to a living body, amyloid which is deposited in biological tissues such as brain, heart, lung, digestive tract, blood vessel, liver, pancreas, kidney, joint and bone can be imaged, and it is useful for imaging amyloid deposition in biological tissues difficult in biopsy collection, for example, brain, heart, lung, pancreas, bone and joint.

## EXAMPLE

Hereinafter, the present invention is described below in more detail by way of Examples, Comparative Examples and Reference Examples. However, these Examples never limit the scope of the present invention.

In the following Examples, the names of the individual compounds used in the experiment are defined as shown in Table 1.

Table 1

| Compound name | Common name |
|---|---|
| Compound 1 | 2-[4-(2-hydroxyethoxy)phenyl]-6-[$^{123}$I]iodoimidazo[1,2-a]pyridine |
| Compound 2 | 2-(4-ethoxyphenyl)-6-[$^{123}$I]iodoimidazo[1,2-a]pyridine |
| Compound 3 | 2-[4-(2-hydroxyethoxy)phenyl]-6-iodoimidazo[1,2-a]pyridine |
| Compound 4 | 2-[3-(2-hydroxyethoxy)phenyl]-6-[$^{123}$I]iodoimidazo[1,2-a]pyridine |

(continued)

| Compound name | Common name |
|---|---|
| Compound 5 | 2-[3-(2-hydroxyethoxy)phenyl]-6-iodoimidazo[1,2-a]pyridine |
| Compound 6 | 2-[4-(3-hydroxypropoxy)phenyl]-6-[$^{123}$I]iodoimidazo[1,2-a]pyridine |
| Compound 7 | 2-[4-(3-hydroxypropoxy)phenyl]-6-iodoimidazo[1,2-a]pyridine |
| Compound 8 | 2-(4-ethoxyphenyl)-6-iodoimidazo[1,2-a]pyridine |
| Compound 9 | 6-tributylstannyl-2-[4-(2-hydroxyethoxy)phenyl]imidazo[1,2-a]pyridine |

Example 1: Synthesis of 2-[4-(2-hydroxyethoxy)phenyl]-6-iodoimidazo[1,2-a]pyridine (non-radioactive iodinatedform)

50 mL of ethyl acetate was added to 28.17 g (corresponding to 126 mmol) of cupric bromide to obtain a suspension, to which a solution of 8.18 g (corresponding to 60.0 mmol) of 4 -hydroxyacetophenone in a mixed solution of 50 mL of ethyl acetate and 50 mL of chloroform was added. Then, the resulting mixture was heated under reflux. After 5 hours, the reaction mixture was cooled down to room temperature and filtered. The resulting filtrate was concentrated under reduced pressure. The residue was dissolved in ethyl acetate and
subjected to decoloring operation with addition of active charcoal. Then, the resulting solution was filtered and concentrated. The resulting crude product was purified by flash silica gel column chromatography (elution solvent: chloroform/methanol = 20/1), and recrystallized from ethyl acetate/petroleum ether, to obtain 7.25 g (corresponding to 33.7 mmol) of 2-bromo-4 - hydroxyacetophenone (Fig. 1, Step 1).

441 mg (corresponding to 2.0 mmol) of 2-bromo-4 - hydroxyacetophenone and 449 mg (corresponding to 2.0 mmol) of 2-amino-5-iodopyridine were dissolved in 15 mL of acetonitrile. The resulting solution was heated under relux in an oil bath at 110˚C for 5 hours. After the completion of the reaction, the reaction solution was cooled down to room temperature, and precipitates were filtered and recovered. The precipitates were washed with acetonitrile and dried under reduced pressure. The resulting crude crystals were suspended in a mixed solution of 10 mL of water and 10 mL of methanol. Then, about 10 mL of a saturated sodium hydrogencarbonate solution was added thereto, and the mixture was sonicated for 5 minutes using an ultrasonic washing machine. Precipitates were filtered and recovered from the resulting mixture, sufficiently washed with water, and dried under reduced pressure, to obtain 526 mg (corresponding to 1.56 mmol) of 2-(4 -hydroxyphenyl)-6-iodoimidazo[1,2-a]pyridine (Fig. 1, Step 2).

Separately, 2.50 g (corresponding to 20.0 mmol) of 2-bromoethanol and 2.72 g (corresponding to 40.0 mmol) of imidazole were dissolved in 10 mL of dimethylformamide (DMF), and cooled to 0˚C. Then, 5.50 g (corresponding to 20.0 mmol) of t-butyldiphenylchlorosilane (TBDPSCl) was added thereto. After the reaction mixture was stirred at room temperature for 18 hours, a saturated sodium chloride aqueous solution was added, and extracted three times with ethyl acetate. The combined ethyl acetate layers were dried over anhydrous sodium sulfate, and concentrated under reduced pressure. The resulting crude product was purified by silica gel column chromatography (elution solvent: hexane/ethyl acetate = 10/1) to obtain 7.04 g (corresponding to 19.4 mmol) of 1-bromo-2-(t-butyldiphenylsiloxy)ethane (Fig. 1, Step 3).

200 mg (corresponding to 0.595 mmol) of 2-(4 - hydroxyphenyl)-6-iodoimidazo[1,2-a]pyridine was dissolved in 3.0 mL of dimethylformamide, and 247 mg (corresponding to 1.79 mmol) of potassium carbonate was added thereto. Then, 259 mg (corresponding to 0.714 mmol) of 1-bromo-2-(t-butyldiphenylsiloxy)ethane was added thereto. After the reaction mixture was stirred at 90˚C for 2 hours, a saturated sodium chloride aqueous solution was added, and extracted three times with ethyl acetate. The combined ethyl acetate layers were dried over anhydrous sodium sulfate, and concentrated under reduced pressure. The resulting crude product was purified by silica gel column chromatography (elution solvent: hexane/ethyl acetate = 2/1) to obtain 368 mg (corresponding to 0.595 mmol) of 2-[4 -(2 -t-butyldiphenylsiloxyethoxy)phenyl]-6-iodoimidazo[1,2-a]pyridine (Fig. 1, Step 4).

368 mg (corresponding to 0.595 mmol) of 2-[4-(2-t-butyldiphenylsiloxyethoxy)phenyl]-6-iodoimidazo[1,2-a]pyridine was dissolved in 1.0 mL or tetrahydrofuran (THF), and 0.70 mL of a 1.0 mol/L solution in tetrahydrofuran of tetrabutylammoniumfluoride (TBAF) was added thereto. After the reaction mixture was stirred at room temperature for 2 hours, ammonium chloride aqueous solution was added, followed by addition of 5.0 mL of water and 2.0 mL of acetonitrile. Then precipitates were filtered. The filtered precipitates were washed with water and acetonitrile in this order, to obtain 226 mg (corresponding to 0.595 mmol) of 2-[4 -(2 - hydroxyethoxy)phenyl]-6-iodoimidazo[1,2-a]pyridine (Fig. 1, Step 5).

The NMR measurement results of the resulting 2-[4-(2-hydroxyethoxy)phenyl]-6-iodoimidazo[1,2-a]pyridine (internal standard: tetramethylsilane) are shown below.

NMR apparatus employed: JNM-ECP-500 (manufactured by Japan Electron Optics Laboratory Co., Lid. (JEOL))
$^{1}$H-NMR (solvent: dimethylsulfoxide-d6; resonance

frequency: 500 MHz): δ 8.95 (s, 1H), 8.27 (s, 1H), 7.87 (d, J = 8.7 Hz, 2H), 7.54-7.46 (m, 2H), 7.04 (d, J = 8.7 Hz, 2H), 4.04 (t, J = 4.6 Hz, 2H), 3.73 (t, J = 4.6 Hz, 2H).

$^{13}$C-NMR (solvent: dimethylsulfoxide-d6, resonance frequency: 125 MHz): δ 158.9, 143.0, 142.4, 133.5, 131.5, 127.1, 124.4, 116.7, 114.8, 108.1, 76.7, 69.5, 59.4.

Example 2: Synthesis of 6-tributylstannyl-2-[4-(2-hydroxyethoxy)phenyl]-imidazo[1,2-a]pyridine

100 mg (corresponding to 0.263 mmol) of 2-[4-(2-hydroxyethoxy)phenyl]-6-iodoimidazo[1,2-a]pyridine obtained in Example 1 was dissolved in 4.0 mL of dioxane, and 2.0 mL of triethylamine was added thereto. Then, 0.20 mL (corresponding to 0.39 mmol) of bis(tributyltin) and 20.1 mg (a catalytic amount) of tetrakis-triphenylphosphine palladium were added thereto. After the reaction mixture was stirred at 90°C for 21 hours, the solvent was distilled off under reduced pressure. The residue was purified by flash silica gel column chromatography (elution solvent: hexane/ethyl acetate = 1/2), to obtain 75.3 mg (corresponding to 0.139 mmol) of 6-tributylstannyl-2-[4-(2-hydroxyethoxy)phenyl]imidazo[1,2-a]pyridine (Fig. 2, Step 1).

The NMR measurement results of the resulting 6-tributylstannyl-2-[4-(2-hydroxyethoxy)phenyl]imidazo[1,2-a]pyridine (internal standard: tetramethylsilane) are shown below.

NMR apparatus employed: JNM-ECP-500 (manufactured by Japan Electron Optics Laboratory Co., Ltd. (JEOL)) $^{1}$H-NMR (solvent: chloroform-d1; resonance frequency: 500 MHz): δ 7.98 (s, 1H), 7.89 (d, J = 8.7 Hz, 1H), 7.75 (s, 1H), 7.56 (d, J = 8.7 Hz, 1H), 7.15 (d, J = 8.7 Hz, 1H), 6.98 (d, J = 8.7 Hz, 1H), 4.13 (t, J = 4.6 Hz, 2H), 3.99 (t, J = 4.6 Hz, 2H), 2.63 (s, 3H), 1.64-1.51 (m, 6H), 1.36 (sextet, J = 7.3 Hz, 6H), 1.19-1.06 (m, 6H), 0.92 (t, J = 7.3 Hz, 9H).

$^{13}$C-NMR (solvent: chloroform-d1, resonance frequency: 125 MHz): δ 158.6, 145.7, 145.0, 131.2, 130.0, 127.4, 127.2, 121.9, 116.9, 114.8, 106.4, 69.3, 61.4, 29.0, 27.3, 13.7, 9.8.

Example 3: Synthesis of 2-[4-(2-hydroxyethoxy)phenyl]-6-[$^{123}$I]iodoimidazo[1,2-a]pyridine

To 60 μL of a solution of 6-tributylstannyl-2-[4-(2-hydroxyethoxy)phenyl]imidazo[1,2-a]pyridine (concentration: 1 mg/mL) in a mixed solution of methanol/dimethylsulfoxide (mixing ratio: 9/1), 150 μL of 1 mol/L hydrochloric acid, 15 μL of 1 mmol/L sodium iodide, 250 μL of [$^{123}$I] sodium iodide of 274 MBq and 15 μL of 10 % (w/v) hydrogen peroxide were added. After the mixed solution was left to stand at 50°C for 10 minutes, it was subjected to HPLC under the following conditions, to obtain a fraction of 2-[4-(2 -hydroxyethoxy)phenyl]-6-[$^{123}$I]iodoimidazo[1,2-a]pyridine.

HPLC conditions:

Column: Phenomenex Luna C18 (trade name; manufactured by Phenomenex Co.; size: 4.6 x 150 mm)
Mobile phase: 0.1 % trifluoroacetic acid in water/0.1 % trifluoroacetic acid in acetonitrile = 80/20 to 0/100 (17 minutes)
Flow rate: 1.0 mL/min.
Detector: Ultraviolet visible absorptiometer (Detection wavelength: 282 nm) and radioactivity counter (manufactured by raptest: type STEFFI)

10 ml of water was added to the fraction. The resulting solution was passed through a reversed phase column (trade name: Sep-Pak (registered trademark) Light C8 Cartridges manufactured by Waters: the packed amount of the packing agent: 145 mg) so that the column adsorbs and collects 2-[4 -(2 -hydroxyethoxy)phenyl]-6-[$^{123}$I]iodoimidazo [1,2-a]pyridine. The column was rinsed with 1 mL of water, and then 1 mL of diethyl ether was passed therethrough to elute 2-[4 -(2 - hydroxyethoxy)phenyl]-6-[$^{123}$I]iodoimidazo[1,2-a]pyridine. The amount of radioactivity of the obtained compound was 22 MBq at the end of synthesis. Further, the TLC analysis was conducted under the following conditions, and as a result, the radiochemical purity of the compound was 97%.

TLC analysis conditions:
TLC plate: Silica Gel 60 $F_{254}$ (trade name; manufactured by Merck & Co., Inc.)
Mobile phase: Chloroform/methanol/triethylaiid.ne = 100/1/2 Detector: Rita Star (trade name; manufactured by raytest)

Example 4: Synthesis of 2 -(4, -ethoxyphenyl) -6-iodoimidazo[1,2-a]pyridine (non-radioactive iodinated form)

30 mL of ethyl acetate was added to 2.72 g (corresponding to 12.2 mmol) of cupric bromide to obtain a suspension, to which 1.00 g (corresponding to 6.09 mmol) of 4 -ethoxyacetophenone was added. Then, the mixture was heated under reflux. After 3 hours, the reaction mixture was cooled down to room temperature and filtered. Then, the resulting filtrate was concentrated under reduced pressure. The residue was dissolved in ethyl acetate and concentrated. The resulting crude produce was purified by silica gel column chromatography (elution solvent: a hexane/ethyl acetate = 10/1), to obtain 1.20 g (corresponding to 4.94 mmol) of 2-bromo-4 - ethoxyacetophenone (Fig. 3, Step 1).

1.20 g (corresponding to 4.94 mmol) of 2-bromo-4 - ethoxyacetophenone and 1.09 g (corresponding to 4.95

mmol) of 2-amino-5-iodopyridine were dissolved in 20 mL of acetonitrile. The resulting solution was heated under reflux in an oil bath at for 1.5 hours. After the completion of the reaction, the reaction solution was cooled down to room temperature, and precipitates were filtered. Then, the precipitates were washed with acetonitrile and dried under reduced pressure. The resulting crude crystals were suspended in a mixed solution of 10 mL of water and 5 mL of methanol. Then, about 20 mL of a saturated sodium hydrogencarbonate solution was added thereto, and the mixture was sonicated for 10 minutes using an ultrasonic washing machine. Precipitates were filtered and recovered from the resulting mixture, sufficiently washed with water, and dried under reduced pressure, to obtain 1.64 g (corresponding to 4.50 mmol) of 2-(4 -ethoxyphenyl)-6-iodoimidazo[1,2-a]pyridine (Fig. 3, Step 2).

The NMR measurement results of the resulting 2-(4 - ethoxyphenyl)-6-iodoimidazo[1,2-a]pyridine (internal standard: tetramehtylsilane) are shown below.

NMR apparatus employed: JNM-ECP-500 (manufactured by Japan Electron Optics Laboratory Co., Ltd. (JEOL)) $^{1}$H-NMR (solvent: dimethylsulfoxide-de; resonance frequency: 500 MHz): δ 9.06 (s, 1H), 8.38 (s, 1H), 7.86 (d, J = 8.7 Hz, 2H), 7.77-7.57 (m, 2H), 7.06 (d, J = 8.7 Hz, 2H), 4.10 (q, J = 6.9 Hz, 2H), 1.36 (t, J = 6.9 Hz, 3H).

$^{13}$C-NMR (solvent: dimethylsulfoxide-d6, resonance frequency: 125 MHz): δ 159.3, 141.1, 140.3, 135.9, 132.0, 127.3, 122.1, 115,3, 114.9, 108.5, 78.6, 63.2, 14.5.

Example 5: Synthesis of 6-tributylstannyl-2-(4ethoxyphenyl)imidazo[1,2-a]pyridine

364 mg (corresponding to 1.00 mmol) of 2-(4 ethoxyphenyl)-6-iodoimidazo[1,2-a]pyridine obtained in Example 4 was dissolved in 4.0 mL of dioxane, and 2 mL of triethylamine was added thereto. Then, 0.76 mL (corresponding to 1.5 mmol) of bis(tributyltin) and 76.3 mg (a catalytic amount) of tetrakis-triphenylphosphine palladium were added thereto. After the reaction mixture was stirred at 90°C for 23 hours, the solvent was distilled off under reduced pressure. The residue was purified by flash silica gel column chromatography (elution solvent: hexane/ethyl acetate = 5/1), to obtain 331 mg (corresponding to 0.628 mmol) of 6-tributylstannyl-2-(4 -ethoxyphenyl)imidazo[1,2-a]pyridine (Fig. 4, Step 1).

The NMR measurement, results of the resulting 6-tributylstannyl-2-(4 -ethoxyphenyl)imidazo[1,2-a] pyridine (internal standard: tetramehtylsilane) are shown below.

NMR apparatus employed: JNM-ECP-500 (manufactured by Japan Electron Optics Laboratory Co., Ltd, (JEOL)) $^{1}$H-NMR (solvent: chloroform-dl; resonance frequency: 500 MHz) : δ 7.96 (s, 1H), 7.88 (d, J = 8.7 Hz, 2H), 7.74 (s, 1H), 7.58 (d, J = 8.7 Hz, 1H), 7.14 (d, J = 6.7 Hz, 1H), 6.96 (d, J = 8.7 Hz, 2H), 4.07 (q, J = 6.9 Hz, 2H), 1.63-1.49 (m, 6H), 1.43 (t, J = 6.9 Hz, 3H), 1.39-1.31 (m, 6H), 1.18-1.04 (m, 6H), 0.90 (t, J = 7.3 Hz, 9H).

$^{13}$C-NMR (solvent: chloroform-d1, resonance frequency: 125 MHz): δ 159.0, 145.7, 145.2, 131.2, 130.1, 127.4, 126.7, 121.9, 117.0, 114.8, 106.4, 63.6, 29.1, 27.4, 15.0, 13.8, 9.9.

Example 6: Synthesis of 2-(4-ethgxyphenyl)-6-[$^{125}$I]iodoimidaz[1,2-a]pyridine

To 60 μL of a solution of 6-tributylstannyl-2-(4-ethoxyphenyl)imidazo[1,2-a]pyridine (concentration: 1 mg/mL) in a mixed solution of methanol/dimethylsulfoxide (mixing ratio: 9/1), 90 μL of 2 mol/L hydrochloric acid, 15 μL of 1 mmol/L sodium iodide, 100 μL of [$^{123}$I] sodium iodide of 436 HBq and 15 μL of 10 % (w/v) hydrogen peroxide were added. After the mixed, solution was left to stand at 50°C for 10 minutes, it was subjected to HPLC under the following conditions to obtain a fraction of 2-(4 -ethoxyphenyl)-6-[$^{123}$I]iodoimidazo[1,2-a]pyridine

HPLC conditions:

Column: Phenomenex Luna C18 (trade name; manufactured by Phenomenex Co.; size: 4.6 x 150 mm)
Mobile phase: 0.1 % trifluoroacetic acid In water/0.1 % trifluoroacetic acid in acetonitrile = 80/20 to 0/100 (17 minutes)
Flow rate: 1.0 mL/min.
Detector: Ultraviolet visible absorptiometer (Detection wavelength: 282 nm) and radioactivity counter (manufactured by raytest: type STEFFI)

10 ml of water was added to the fraction. The resulting solution was passed through a reversed phase column (trade name: Sep-Pak (registered trademark) Light C8 Cartridges manufactured by Waters: the packed amount of the packing agent: 145 mg) so that the column adsorbs and collects 2-(4 -ethoxypenyl)-6-[$^{123}$I]iodoimidazo[1,2-a]pyridine. The column was rinsed with 1 mL of water, and then 1 mL of diethyl ether was passed therethrough to elute 2-(4 -ethoxyphenyl)-6-[$^{123}$I]iodoimidazo[1,2 a]pyridine. The amount of radioactivity of the obtained compound was 88 MBq at the end of synthesis. Further, the TLC analysis was conducted under the following conditions, and as a result, the radiochemical purity of
the compound was 98%.

TLC analysis conditions:

TLC plate: Silica Gel 60 $F_{254}$ (trade name, manufactured by Mercy & Co., Inc.)
Mobile phase: Chloroform/methanol/triethylamine = 100/1/2 Detector: Rita Star (trade name; manufactured by raytest)

Reference Example 1: Synthesis of [$^{123}$I]-IMPY

[$^{123}$I]-IMPY was synthesized in accordance with the following steps for use in Comparative Examples for evaluations on measurement of $logP_{octanol}$ and accumulations in brain.

In accordance with the method described in a literature (Zhi-Ping Zhuang et al., J. Med. Chem, 2003, 46, p. 237-243), 6-tribütyistannyl-2-[4 -(N,N-dimethylamino)phenyl]imidazo[1,2-a]pyridine was synthesized, and dissolved in methanol (concentration: 1mg/mL). To 53 μL of the resulting solution, 75 μL of 1 mol/L hydrochloric acid, 60-70 μL of [$^{123}$I]sodium iodide of 224-253 MBq, 10 μL of a 1 mmol/L sodium iodide solution and 15 μL of 10% (w/v) hydrogen peroxide were added. After the mixed solution was left to stand at 50˚C for 10 minutes, the solution was subjected to HFLC under the same conditions as in Example 3, to obtain a fraction of [$^{123}$I]-IMPU.

10 ml of water was added to the fraction. The resulting solution was passed through a reversed phase column (trade name: Sep-Pak (registered trademark) Light C8 Cartridges manufactured by Waters; the packed amount of the packing agent: 145 mg), so that the column adsorbs and collects the [$^{123}$]-IMPY, The column was rinsed with 1 mL of water, and then 1 mL of diethyl ether was passed therethrough, to elute [$^{123}$I]-IMPY. The obtained radioactivity was 41-57 MBq at the end of synthesis. Further, the TLC analysis was conducted under the same condition as described in Example 3, and as a result, the radiochemical purity of the compound was 93%.

Example 7, Comparative Example 1 to 3: Measurement of affinity with amyloid

Affinity of the present compounds with amyloid was examined by the following *in vitro* binding tests.

(1) $A\beta_{1-42}$ (manufactured by Wako) was dissolved in phosphate buffer (pH 7.4) and shaken at 37˚C for 72 hours, to obtain 1 mg/mL of a suspension (hereinafter referred to as amyloid suspension in this Example) of aggregated Aβ (hereinafter referred to as amyloid in this Example).

(2) According to the method described in a literature (Naiki, H., et al.. Laboratory Investigation 74, p,374-383 (1996)), the amyloid suspension was subjected to qualitative experiment based on fluorescence spectrophotometric method using Thioflavin T (manufactured by Fluka) to confirm that the aggregated Aβ obtained in (1) was amyloid (measurement conditions: excitation wavelength of 446 nm, and emission wavelength of 490 nm).

(3) According to the method described in a literature (Wang, Y., et al., J. Labeled Compounds Radiopharmaceut. 44, S239 (2001)), [$^{125}$I]2-(3 -iodo-4 - aminophenyl)benzothiazole (hereinafter referred to as [$^{125}$I]3 -I-BTA-0) was prepared from a labeling precursor 2-(4 -aminophenyl)benzothiazole, and dissolved in ethanol. As Congo Red, Thioflavin T and 6-methyl-2-[4 -(N,N-dimethylamlno)phenyl]benzothlazole (hereinafter referred to as 6-Me-BTA-2), commercially available reagents were weighed and used as they were.

(4) IMPY was synthesized according to the method described in a literature (Zhuang, Z.P., et al., J. Med. Chem. 46, 237 (2003)).

(5) Each compound for evaluation or ethanol solution thereof, an ethanol solution of [$^{125}$I]3 -I-BTA-0 prepared above in (3) and an amyloid suspension prepared above in (1) were dissolved in 1 % bovine serum albumin-containing phosphate buffer (pH 7.4), and samples having final concentrations of each compound for evaluation, [$^{125}$I]3 - I-BTA-0 and amyloid shown in Table 2 respectively was prepared.

Table 2: Final concentrations of each compound in sample solutions

| Experiment | Compound for evaluation | Concentration of compound for evaluation | [$^{125}$I]3 -I-BTA-0 concentration | Amyloid |
|---|---|---|---|---|
| Comparative Example 1 | Congo Red | Each concentration of 0, 0.001, 10, 100, 1000 nmo1/L | 400 pmol/L | 1 μmol/L. |
| Comparative Example 2 | Thioflavin T | | | |
| Comparative Example 3 | IMPY | | | |
| Example 7 | Compound 3 | | | |

(6) Each sample solution prepared above in (5) was filled in each well (about 0.3 mL in volume) of a 96-well microplate. The microplate filled with the sample solutions was shaken at a given rats (400 rpm) at 22˚C for 3 hours. Then, each sample solution was filtered through a glass fiber filter (trade name: Mulutiscreen™-FC, manufactured by Millipore), to separate the [$^{125}$I]3-I-BTA-0 attached to amyloid from the free [$^{125}$I]3 -I-BTA-0.

(7) The glass fiber filter used for the filtration of each sample solution was washed with 1 % bovine serum albumin-containing phosphate buffer (pH 7.4) (0.5 mL x 5), and radioactivity off the glass fiber filter was measured with an autowell gamma system (manufactured by Aloka, Type: ARC-301B) (hereinafter, A denotes the radioactivity level in a sample with zero (0) concentration of each compound for evaluation, and B denotes the radioactivity level in a sample with 0.001 nmol/L or higher concentration of each compound for evaluation).

(8) Separately, a solution containing 15 μmol/L of 6-Me-BTA-2, 400 pmol/L of [$^{125}$I]3 -I-BTA-0 and 1 μmol/L of amyloid were prepared and subjected to the same procedures as described above in (7) and (8) to measure a radioactivity level. The measured radioactivity level was defined as the background radioactivity level, and used in the calculation of the inhibition ratio (hereinafter referred to as BG).

(9) Using the radioactivity levels measured above in (7) and (8), the inhibition ratio was determined by the following formula (1) .

$$\frac{B-BG}{A-BG}\times 100 \qquad (\%) \qquad (1)$$

A graph in which values converted by probit transformation from the obtained inhibition ratios were plotted relative to logarithms of concentrations of compounds for evaluation was prepared to obtain an approximate straight line by the least square method. Using the line, a 50 % inhibition concentration of each compound for evaluation (hereinafter referred to as $IC_{50\%}$ value) was determined. Using the value as an indicator, affinity of each compound for evaluation with amyloid was evaluated.

$IC_{50\%}$ value of each compound for evaluation is shown in Table 3. Compounds 3 showed $IC_{50\%}$ values of less than 100 and had significantly higher affinity with amyloid than Congo Red and Thioflavin T which are generally known to have affinity with amyloid. The results show that Compounds 3 has good affinity with amyloid like IMPY.

Table 3: $IC_{50\%}$ values of the present compounds

| Experiment | Compound for evaluation | $IC_{50\%}$ values (nmol/L) |
|---|---|---|
| Comparative Example 1 | Congo Red | >1000 |
| Comparative Example 2 | Thioflavin T | > 1000 |
| Comparative Example 3 | IMPY | 25.8 |
| Example 7 | Compound 3 | 66.9 |

Example 8 to 9, Comparative Example 4: Measurement of partition coefficient based on the octanol extraction method

Partition coefficients based on the octanol extraction method (hereinafter referred to as $\log P_{octanol}$) were measured, which are generally known as an indicator of permeability of compounds through the blood-brain barrier (hereinafter referred to as BBB).

A diethyl ether solution of Compound 1 prepared in Example 3 (Example 8), a diethyl ether solution of Compound 2 prepared in Example 6 (Example 9), and a diethyl ether solution of [$^{123}$I]-IMPY prepared in Reference Example 1 (Comparative Example 4) were each diluted with 10 mg/mL ascorbic acid-containing physiological saline solution, and adjusted to a radioactive concentration of 20-30 MBq/mL. To 2 mL of octanol, 10 $\mu$L each of the prepared sample solutions was added, 2 mL of 10 mmol/L phosphate buffer (pH 7.4) was added, followed by stirring for 30 seconds. After the mixture was centrifuged with a low-speed centrifuge (2000 rpm x 60 min.), the octanol layer and the water layer were sampled each in an amount of 1 mL, and subjected to measurement of radioactivity count with an autowell gamma system (Type: ARC-301B, manufactured by Aloka). Using the obtained radioactivity count, $\log P_{octanol}$ was calculated in accordance with the equation (2).

$$\log P_{octanol} = \log_{10}\left(\frac{\text{Radioactivity count of octanol layer}}{\text{Radioactivity count of water layer}}\right) \cdots (2)$$

The results are shown in Table 4. All the compounds showed $\log P_{octanol}$ values between 1 and 3. It is known that compounds permeable to BBB show a $\mathrm{loqP}_{octanol}$ value between 1 and 3 (Douglas D. Dischino et al., J. Nucl. Med., (1983), 24, p.1030-1038). From the above results, it is implied that both compounds have a BBB permeability like IMPY.

Table 4: $\log P_{octanol}$ value of the present compound

| Experiment | Compound value | $\log P_{octanol}$ value |
|---|---|---|
| Comparative Example 4 | [$^{123}$I]-IMPY | 1.9 |
| Example 8 | Compound 1 | 1.8 |
| Example 9 | Compound 2 | 2.1 |

Example 10 to 11, Comparative Example 5: Measurement of transferability into brain and clearance

Using Compound 1 (Example 10) and Compound 2 (Example 11), a time course change of radioactive accumulation in brain of male Wistar rats (7-week old) was measured.

A diethyl ether solution of Compound 1 (Example 10) prepared in Example 3, a diethyl ether solution of Compound 2 (Example 11) prepared in Example 6 and a diethyl ether solution of [$^{123}$I]-IMPY (Comparative Example 5) prepared in Reference Example 1 were each diluted with 10 mg/mL ascorbic acid-containing physiological saline solution to adjust to a radioactive concentration of 8-12 MBq/mL, 0.05 mL each of the prepared sample solutions was injected under thiopental anesthesia into the tail vein of the rats. The rats were sacrificed by bleeding from abdominal artery, and brains were removed and subjected to measurement of mass of brains and further subjected to measurement of radioactivity (hereinafter referred to as A in this Example) with a single channel analyzer (detector type: SP-20 manufactured by OHYO TOKEN KOGYO Co., Ltd.) 2, 5, 30 and 60 minutes after the injection. Further, the radioactivity level of the rest of the whole body was measured in the same manner as above (hereinafter referred to as B in this Example). Using these measurement results, radioactive distribution per unit weight of brain (%ID/g) at the respective time points were calculated in accordance with the following formula (3).
Three were used for the experiment at the respective time points.

$$\%ID/g = \frac{A}{B \times brain\ weight} \times 100 \quad \cdots (3)$$

The results are shown in Table 5. As shown in Table 5, Compounds 1 and 2 showed a significant radioactive accumulation like [$^{123}$I]-IMPY at the time point of two minutes after the injection, and then showed a tendency to rapidly clear away in 60 minutes. These results suggest that both Compounds 1 and 2 possess excellent transferability to brain and rapid clearance from brain like [$^{123}$I]-IMPY.

Table 5: Radioactive distribution in brain of the present compound after intravenous injection (rats)

| Compound | | Radioactive distribution per unit weight (%ID/g) | | | |
|---|---|---|---|---|---|
| | | After 2 min. | After 5 min. | After 30 min. | After 60 min. |
| Example 10 | Compound 1 | 0.90 | 0.52 | 0.06 | 0.01 |
| Example 11 | Compound 2 | 0.89 | 0.66 | 0.13 | 0.04 |
| Comparative Example 5 | $^{123}$I-IMPY | 1.19 | 0.97 | 0.23 | 0.09 |

Comparative Example 6: ex *vivo* autoradiogram of $^{123}$I-IMPY using rats of amyloid injected model

(1) $A\beta_{1-40}$ (manufactured by Peptide Institute, INC.) was dissolved in phosphate buffer (pH 7.4) and shaken at. 37˚C for 72 hours, to obtain a 1 mg/mL suspension of aggregated Aβ (hereinafter referred to as amyloid suspension in this Example).

(2) 2.5 μL (corresponding to 25 μg) of the amyloid suspension was injected into an amygdaloid nucleus on one side of a male rat (7-week old). As a control, 2.5 μL of a phosphate buffered physiological saline solution (pH 7.4) was injected into an amygdaloid nucleus on the other side of the rat. The rats were examined 1 day after the injection of the amyloid suspension and the phosphate buffered physiological saline solution (pH 7.4).

(3) [$^{123}$I]-IMPY was dissolved in a 10 mg/mL ascorbic acid-containing physiological saline solution to obtain a sample solution (29 MBq/mL in radioactivity concentration in the sample solution). This solution was injected under thiopental anesthesia into the rat through the tail vein (dosage: 0.5 mL, dosed radioactivity: 14.5 MBq equivalent).

(4) Brain was removed 60 minutes after the injection to prepare a brain slice of 10 μm in thickness with a microtome (type: CM3050S, manufactured by LEICA). The brain slice was exposed to an imaging plate for 20 hours, and then image analysis was carried out by use of a Bio-imaging Analyzer (type: BAS-2500; manufactured by FUJIFILM Corporation).

(5) After the completion of the image analysis using the Bio-imaging Analyzer, pathological staining with Thioflavin T was carried out to perform imaging by use of a fluorescence microscope (manufactured by NIKON Corporation; type: TE2000-U model; excitation wavelength: 400-440 nm; detection wavelength: 470 nm). Thus, it was confirmed that amyloid was deposited on the slice (Fig. 5b).

Fig. 5 shows images by autoradiogram and Thioflavin T staining of the brain slice of the rat to which amyloid was injected intracerebrally. As shown in this figure, a marked accumulation of radioactivity was observed in the amygdaloid nucleus on the side to which the amyloid suspension was injected, but also non-specific accumulation was observed in white matter where amyloid was not injected.

Example 12: Confirmation of imaging of amyloid in brain

The following experiment was carried out in order to examine whether amyloid in brain can be imaged by the compound of the present invention.

(1) $A\beta_{1-42}$ (manufactured by Wako) was dissolved in phosphate buffer (pH 7.4) and shaken at 37°C for 72 hours, to obtain a 1 mg/mL suspension of aggregated Aβ (hereinafter referred to as amyloid suspension in the Examples).

(2) 2.5 μL (corresponding to 25 μg) of the amyloid suspension was injected into an amygdaloid nucleus on one side of a male Wistar rat (7-week old). As a control, 2.5 μL of a phosphate buffered physiological saline solution (pH 7.4) was injected at amygdaloid nucleus on the other side of the rat. The were examined 1 day after the injection of the amyloid suspension and the phosphate buffered physiological saline solution (pH 7.4).

(3) Compound 1 was dissolved in a 10 mg/mL ascorbic acid-containing physiological saline solution to obtain a sample solution (22 MBq/mL in radioactivity concentration in the sample solution). This solution was injected under thiopental anesthesia into the rat through the tail vein (dosage: 0.5 mL, dosed radioactivity: 11-13 MBq equivalent).

(4) Brain was removed 60 minutes after the injection to prepare a brain slice of 10 μm in thickness with a microtome (type: CM3050S, manufactured by LEICA), The brain slice was exposed to an imaging plate for 20 hours, and then image analysis was carried out by use of a Bio-imaging Analyzer (type: BAS-2500; manufactured by FUJIFILM Corporation).

(5) After the completion of the image analysis using the Bio-imaging Analyzer, pathological staining with Thioflavin T was carried out to perform imaging by use of a fluorescence microscope (manufactured by NIKON Corporation; type: TE2000-U model; excitation wavelength: 400-440 nm; detection wavelength: 470 nm). Thus, it was confirmed that amyloid was deposited on the slice (Fig. 6b).

Fig. 6 shows images by autoradiogram and Thioflavin T staining of the brain slice of the rat to which amyloid was injected intracerebrally. As shown in this figure, a marked accumulation of radioactivity was observed in the amygdaloid nucleus on the side to which the amyloid suspension was Injected. On the other hand, no significant, accumulation of radioactivity was observed in the amygdaloid nucleus on the side to which the physiological saline solution was injected, compared with the other sites. On the autoradiogram, little accumulation of radioactivity was observed at sites other than the sites to which amyloid was injected. From the result of Thioflavin T staining, it was confirmed that amyloid was present in the site where radioactivity is accumulated (Fig. 6b).

Thus, Compound 1 showed little radioactive accumulation at the sites other than amyloid injected sites, and showed little non-specific binding to the white matter observed in [$^{123}$I]-TMPY. These results suggest that Compound 1 possesses an excellent capability of imaging amyloid in the total autoradiogram image. These results also suggest that Compound 1 is a compound that possesses a high specificity to imaging of intracerebral amyloid.

Example 13: Confirmation of imaging of amyloid in brain

The same procedures as in Example 12 were performed except using a solution of Compound 2 in a mg/mL ascorbic acid as a sample solution (the radioactive concentration of the sample solution was 25 MBq/mL).

Fig. 7 shows images by autoradiogram and Thioflavin T staining of the brain slice of the rat to which amyloid was injected intracerebrally. As shown in this figure, a marked accumulation of radioactivity was observed in the amygdaloid nucleus on the side to which the amyloid suspension was injected. From the result off Thioflavin T staining in the site where radioactivity was accumulated, it was confirmed that amyloid was present in the accumulation site. On the other hand, no significant accumulation of radioactivity was observed in the amygdaloid nucleus on the side to which the physiological saline solution was injected, compared with the other sites.

Compound 2 showed some radioactive accumulation in sites other than amyloid injected sites, but the accumulation was highly suppressed as compared to $^{123}$I-IMPY. As a result, the whole image was provided with a high capability of imaging amyloid.

These results suggest that Compound 2 is a compound that possesses a high specificity to imaging of intracerebral amyloid.

Example 14: Synthesis of 2-[3-(2-hydroxyethoxy)phenyl]-6-iodoimidazo[1,2-a]pyridine (non-radioactive iodinated form)

50 mL of ethyl acetate was added to 8.60 g (corresponding to 46. mmol) of cupric bromide to obtain a suspension, to which 2.50 g (corresponding to 22.0 mmol) of 3 -hydroxyacetophenone was added. Then, the resulting mixture was heated under reflux. After 2 hours, the reaction, mixture was cooled down to room temperature and filtered. The resulting filtrate was concentrated under reduced pressure. The residue was dissolved in ethyl acetate and subjected to decoloring operation with addition of active charcoal. Then, the resulting solution was filtered and concentrated. The resulting crude product was purified by flash silica gel column chromatography (elution solvent: hexane/ethyl acetate = 2/1) to obtain 4.42 g (corresponding to 20.6 mmol) of 2-bromo-3 -hydroxyacetophenone (Fig. 8, Step 1).

987 mg (corresponding to 4.45 mmol) of 2-bromo-3-hydroxyacetophenone and 1.00 g (corresponding to 4.55 mmol) of 2-amino-5-iodopyridine were dissolved in 50 mL of acetonitrile. The resulting solution was heated under reflux in an oil bath at 110˚C for 2 hours. After the completion of the reaction, the reaction solution was cooled down to room temperature, and precipitates were filtered and recovered. The precipitates were washed with acetonitrile and dried under reduced pressure. The resulting crude crystals were suspended in a mixed solution of 10 mL of water and 1 mL of methanol. Then, about 10 mL or a saturated sodium hydrogencarbonate solution was added thereto, and the mixture was sonicated for 5 minutes using an ultrasonic washing machine. Precipitates were filtered and recovered from the resulting mixture, sufficiently washed with water, and dried under reduced pressure, to obtain 927 mg (corresponding to 2.76 mmol) of 2-(3 -hydroxyphenyl)-6-iodoimidazo[1,2-a]pyridine (Fig. 8, Step 2).

Separately, 2.50 g (corresponding to 20.0 mmol) of 2-bromoethanol and 2.72 g (corresponding to 40.0 mmol) of imidazole were dissolved in 10 mL of dimethylformamide, and cooled to 0˚C. Then, 5.50 g (corresponding to 20.0 mmol) of t-butyldiphenylchlorosilane was added thereto. After the reaction mixture was stirred at room temperature for 18 hours, a saturated sodium chloride aqueous solution was added, and extracted three times with ethyl acetate. The combined ethyl acetate layers were dried over anhydrous sodium sulfate, and concentrated under reduced pressure. The resulting crude product was purified by silica gel column chromatography (elution solvent: hexane/ethyl acetate = 10/1) to obtain 7.04 g (corresponding to 19.4 mmol) of 1-bromo-2-(t-butyldiphenylsiloxy)ethane (Fig. 8, Step 3).

300 mg (corresponding to 0.893 mmol) of 2-(3-hydrozyphenyl)-6-iodoimidazo[1,2-a]pyridine was dissolved in 5.0 mL of dimethylformamide, and 370 mg (corresponding to 2.68 mmol) of potassium carbonate was added thereto. Then, 357 mg (corresponding to 0.982 mmol) of 1-bromo-2-(t-butyldiphenylsiloxy)ethane was added thereto. After the reaction mixture was stirred at 90˚C for 2 hours, a saturated sodium chloride aqueous solution was added, and extracted three times with ethyl acetate. The combined ethyl acetate layers were dried over anhydrous sodium sulfate, and concentrated under reduced pressure. The resulting crude product was purified by silica gel column chromatography (elation solvent: hexane/ethyl acetate = 3/1) to obtain 477 mg (corresponding to 0.771 mmol) of 2-[3-(2-t-butyldiphenylsiloxyethoxy)phenyl]-6-iodoimidazo[1,2-a]pyridine (Fig. 8, Step 4).

477 mg (corresponding to 0.771 mmol) of 2-[3-(2-t-butyldiphenylsiloxyethoxy)phenyl]-6-iodoimidazo[1,2-a]pyridine was dissolved in 0.98 mL of tetrahydrofuran, and 0.93 mL of a 1.0 mol/L tetrahydrofuran solution of tetrabutylamoniumfluoride was added thereto. After the reaction mixture was stirred at room temperature for 15 minutes, ammonium chloride aqueous solution was added followed by addition of 5.0 mL of water and 2.0 mL of acetonitrile to filter precipitates. The filtered precipitates were washed with water and acetonitrile in this order to obtain 120 mg (corresponding to 0.316 mmol) of 2-[3-(2 -hydroxyethoxy)phenyl]-6-iodoimidazo[1,2-a]pyridine (Fig. 8, Step 5).

The NMR measurement results of the resulting 2-[3 - (2-hydroxyethoxy)phenyl]-6-iodoimldazo[1,2-a]pyrldine (internal standard: tetramehtylsilane) are shown below.

NMR apparatus employed: JNM-ECP-500 (manufactured by Japan Electron Optics Laboratory Co., Ltd. (JEOL))

$^1$H-NMR (solvent: dimethylsulfoxide-d6; resonance frequency: 500 MHz); δ 8.91 (s, 1H), 8.35 (s, 1H), 7.52-7.51 (m, 2H), 7.45 (s, 2H), 7.35 (t, J = 8.2 Hz, 1H), 6.93-6.90 (m, 1H), 4.06 (t, J = 4.6 Hz, 2H), 3.75 (t, J = 4.6 Hz, 2H).

Example 15: Synthesis of 6-tributylstannyl-2-[3-(2-hydroxyethoxy)phenyl]-imidazo[1,2-a]pyridine

70 mg (corresponding to 0.184 mmol) of 2-[3-(2-hydroxyethoxy)phenyl]-6-iodoimidazo[1,2,-a]pyridine obtained in Example 14 was dissolved in 4.0 mL of dioxane, and 2.0 mL of triethylamine was added thereto. Then, 0.20 mL (corresponding to 0.39 mmol) of bis(tributyltih) and 14.0 mg (a catalytic amount) of tetrakis-triphenylphosphine palladium were added thereto. After the reaction mixture was stirred at 90°C for 20 flours, the solvent was distilled off under reduced pressure.
The resid-ae was purified by flash silica get column chromatography (elution solvent: hexane/ethyl acetate = 2/1) to obtain 73.0 mg (corresponding to 0.134 mmol) of 6-tributylstannyl-2-[3-(2-hydroxyethoxy)phenyl]imidazo[1,2-a]pyridine (Fig. 9, Step 1).
The NMR measurement results of the resulting 6-tributylstannyl-2-[3 -(2-hydroxyethoxy)phenyl]imidazoi[1,2-a]pyridine (internal standard: tetramehtylsilane) are shown below.
NMR apparatus employed: JNM-ECP-500 (manufactured by Japan Electron Optics Laboratory Co., Ltd. (JEOL))
$^1$H-NMR (solvent: chloroform-dl; resonance frequency: 500 MHz): δ 7.99 (d, J = 0.9 Hz, 1H), 7.82 (s, 1H), 7.64-7.50 (m, 3H), 7.34-7.31 (m, 1H), 7.18-7.17 (m, 1H), 6.90-6.87 (m, 1H), 4.20 (t, J = 4.3 Hz, 2H), 3.98 (t, J = 4.3 Hz, 2H), 1.69-1.48 (m, 6H), 1.39-1.32 (m, 6H), 1.19-1.05 (m, 6H), 0.91 (t, J = 7.4 Hz, 9H).

Example 16: Synthesis of 2-[3 -(2 -hydroxyethxy)phenyl]-6-[$^{123}$I]iodolmidazo[1,2-a]pyrlaine

To 60 μL of a solution of 6-tributylstannyl-2-[3-(2 -hydroxyethoxy)phenyl]imidazo[1,2-a]pyridine (concentration: 1mg/mL) in a mixed solution of methanol/dimethylsulfoxide = 9/1, 150 μL of 1 mol/L hydrochloric acid, 15 μL of 1 mmol/L sodium iodide, 250 μL of [$^{125}$I]sodiuxn iodide of 274 MBq and 15 μL of 10 % (w/v) hydrogen peroxide were added. After the mixed solution was left to stand at 50°C for 10 minutes, it was subjected to HPLC under the following conditions to obtain a fraction of 2-[3 -(2 -hydroxyethoxy)phenyl]-6-[$^{123}$I]iodoimidazo[1,2-a]pyridine.
HPLC conditions:

Column: Phenomenex Luna C18 (trade name; manufactured by Phenomenex Co.; size: 4.6 x 150 mm)
Mobile phase: 0.1 % trifluoroacetic acid in water/0.1 % trifluoroacetic acid in acetonitrile = 20/80 to 0/100 (17 minutes)
Flow rate: 1.0 mL/min.
Detector: Ultraviolet visible absorptiometer (Detection wavelength: 282 nm) and radioactivity counter (manufactured by raytest: type STEFFI)

10 ml of water was added to the fraction. The resulting solution was passed through a reversed phase column (trade name: Sep-Pak (registered trademark) Light C8 cartridges manufactured by Waters: the packed amount of the packing agent: 145 mg) so that the column adsorbs and collects 2-[3 -(2 -hydroxyethoxy)phenyl]-6-[$^{125}$I]iodoimidazo [1,2-a]pyridine. The column was rinsed with 1 mL of water, and then 1 mL of diethyl ether was passed therethrough to elute 2-[3-(2-hydroxyethoxy)phenyl]-6-[$^{123}$I]iodoimidazo[1,2-a]pyridine. The amount of radioactivity of the obtained compound was 112.9 MBq at the end of synthesis. Further, the TLC analysis was conducted under the following conditions, and as a result, the radiochemical purity of the compound was 97%.
TLC analysis conditions:

TLC plate: Silica Gel 60 $F_{254}$ (trade name; manufactured by Merck & Co., Inc.)
Mobile phase: Chloroform/methanol/triethylamine = 100/1/2 Detector: Rite Star (trade name; manufactured by raytest)

Example 17: Synthesis of 2-[4-(3-hydroxypropoxy)phenyl]-6-iodoimidazo[1,2-a]pyridine (non-radioactive iodinated form)

50 mL of ethyl acetate was added to 28.17 g (corresponding to 126 mmol) of cupric bromide to obtain a suspension, to which a solution of 8.18 g (corresponding to 60.0 mmol) of 4 -hydroxyacetophenone in a mixed solution of 50 mL of ethyl acetate and 50 mL of chloroform was added. Then, the resulting mixture was heated under reflux. After 5 hours, the reaction mixture was cooled down to room temperature and filtered. The resulting filtrate was concentrated under reduced pressure. The residue was dissolved in ethyl acetate and subjected to decoloring operation with addition of active charcoal. Then, the resulting solution was filtered any concentrated. The resulting crude product was purified by flash silica, gel column chromatography (elution solvent: chloroform/methanol = 20/1), and recrystallized

from ethyl acetate/petroleum ether, to obtain 7.25 g (corresponding to 33.7 mmol) of 2-bromo-4 - hydroxyacetophenone (Fig. 10, Step 1).

987 mg (corresponding to 4.55 mmol) of 2-bromo-4 hydroxyacetophenone and 1.00 g (corresponding to 4.55 mmol) of 2-amino-5-iodopyridine were dissolved in 50 mL of acetonitrile. The resulting solution was heated under reflux in an oil bath at 110˚C for 2 hours. After the completion of the reaction, the reaction solution was cooled down to room temperature, and precipitates were filtered and recovered. The precipitates were washed with acetonitrile and dried under reduced pressure. The resulting crude crystals were suspended in a mixed solution of 10 mL of water and 1 mL of methanol. Then, about 10 mL of a saturated sodium hydrogencarbonate solution was added thereto, and the mixture was sonicated for 5 minutes using an ultrasonic washing machine. Precipitates were filtered and recovered from the resulting mixture, sufficiently washed with water, and dried under reduced pressure, to obtain 927 mg (corresponding to 2.76 mmol) of 2-(4 -hydroxyphenyl)-6-iodoimidazo[1,2-a]pyridine (Fig. 10, Step 2).

Separately, 7.0 g (corresponding to 50.4 mmol) of 3-bromo-1-propanol and 6.86 g (corresponding to 101 mmol) of imidazole were dissolved in 50 mL of dimethylformamide, and cooled to 0˚C. Then, 7.59 g (corresponding to 50.4 mmol) of t-butyldimethylichlorosilane was added thereto. After the reaction mixture was stirred at room temperature for 24 hours, it was supplemented with a saturated sodium chloride aqueous solution, and extracted three times with diethyl ether. The combined diethyl ether layers dried over anhydrous magnesium sulfate, and concentrated under reduced pressure. The resulting crude product was purified by vacuum distillation (100˚C, 70 mmHg), to obtain 7.23 g (corresponding to 30.2 mmol) of 1-bromo-3-(t-butyldimethylsiloxy) propane (Fig. 10, Step 3).

2.00 g (corresponding to 5.95 mmol) of 2-(4 - hydroxyphenyl)-6-iodoimidazo[1,2-a]pyridine was dissolved in 30.0 mL of dimethylformamide, and 2.47 g (corresponding to 17.9 mmol) of potassium carbonate was added. Then, 1.51 g (corresponding to 5.95 mmol) of 1-bromo-3-(t-butyldimethylsiloxy)propane was added thereto. After the reaction mixture was stirred at room temperature for 8 days, it was supplemented with a saturated sodium chloride aqueous solution, and extracted three times with ethyl acetate. The combined ethyl acetate layers were dried over anhydrous sodium sulfate, and concentrated under reduced pressure. The resulting crude product was purified by silica gel column chromatography (elution solvent: hexane/ethyl acetate - 1/1) to obtain 1.52 g (corresponding to 2.99 mmol) of 2-[4-(3-t-butyldimethylsiloxypropoxy)phenyl]-6-iodoimidazo[1,2-a]pyridine (Fig. 10, Step 4).

1.52 g (corresponding to 2.99 mmol) of 2-[4-(3-t-butyldimethylsiloxypropoxy)phenyl]-6-iodoimidazo[1,2-a]pyridine was dissolved in 5.0 mL of tetrahydrofuran, and 2.39 mL of a 1.0 mol/L tetrahydrofuran solution of tetrabutylammoniumfluoride was added thereto. After the reaction mixture was stirred at room temperature for 30 minutes, ammonium chloride solution was added followed by the addition of 10 mL of water and 5.0 mL of acetonitrile to filter precipitates. The filtered precipitates were washed with water and acetonitrile in this order, to obtain 1.03 g (corresponding to 2.61 mmol) of 2-[4 -(3 - hydroxypropoxy)phenyl]-6-iodoimidazo[1,2-a]pyridine (Fig. 10, Step 5).

The NMR measurement results of the resulting 2-[4-(3 -hydroxypropoxy)phenyl]-6-iodoimidazo[1,2-a]pyridine (internal standard: tetramethylsilane) are shown below.

NNR apparatus employed: JNH-ECP-500 (manufactured by Japan Electron Optics Laboratory Co., Ltd. (JEOL)) $^1$H-NMR (solvent: dimethylformamide-d6; resonance frequency: 500 MHz): 8.96 (s, 1H), 8.33 (s, 1H), 7.98 (d, J = 8.7 Hs. 2H), 7.46 (s, 2H), 7.06 (d, J = 8.7 Hz, 2H), 4.63 (t, J = 5.0 Hz, 1H), 4.17 (t., J = 6.0 Hz, 2H), 3.72 (dt, J = 5.0, 6.0 Hz, 2H), 1.98 (tt, 7 = 6.0, 6.0 Hz, 2H).

<u>Example 18: Synthesis of 6-tributylstannyl-2-[4-(3-hydroxypropoxy)phenyl]-imidazo[1,2-a]pyridine</u>

50 mL of ethyl acetate was added to 28.17 g (corresponding to 126 mmol) of cupric bromide to obtain a suspension, to which a solution of 8.18 g (corresponding to 60.0 mmol) of 4 -hydroxyacetophenone in a mixed solution of 50 mL of ethyl acetate and 50 mL of chloroform was added. Then, the resulting mixture was heated under reflux. After 5 hours, the reaction mixture was cooled down to room temperature and filtered. The resulting filtrate was concentrated under reduced pressure. The residue was dissolved in ethyl acetate and subjected to decoloring operation with addition of active charcoal. Then, the resulting solution was filtered and concentrated. The resulting crude product was purified by flash silica gel column chromatography (elution solvent: chloroform/methanol = 20/1), and recrystallized from ethyl acetate/petroleum ether, to obtain 7.25 g (corresponding to 33.7 mmol) of 2-bromo-4 - hydroxyacetophenone (Fig. 11, Step 1).

2.15 g (corresponding to 10.0 mmol) of 2-bromo-4 - hydroxyacetophenone and 1.74 g (corresponding to 10.0 mmol) of 2-amino-5-bromopyridine were dissolved in 50 mL of acetonitrile. The resulting solution was heated under reflux in an oil bath at 105 ˚C for 6 hours. After the completion of the reaction, the reaction solution was cooled down to room temperature, and precipitates were filtered and recovered. The precipitates were washed with acetonitrile and dried under reduced pressure. The resulting crude crystals were suspended in a mixed solution of 20 mL of water and 20 mL of methanol. Then, about 25 mL of a saturated sodium hydrogencarbonate solution was added thereto, and the mixture was sonicated for 5 minutes using an ultrasonic washing machine. Precipitates were filtered and recovered from the resulting mixture, sufficiently washed with water, and dried under reduced pressure, to obtain 2.41 g (corresponding

to 8.32 mmol) of 6-bromo-2-(4 - hydroxypheny)imidazo[1,2-a]pyridine (Fig. 11, Step 2).

1.45 g (corresponding to 5.0 mmol) of 6-bromo-2-(4 - hydroxyphenyl)imidazo[1,2-a]pyridine that was sufficiently dried to remove moisture was dissolved in 50 mL of N,N-dimethylformamide, and 2.07 g (corresponding to 15.0 mmol) of potassium carbonate was added thereto. The mixture was supplemented with 680 μL (corresponding to 7.5 mmol) of 3-bromo-1-propanol, and then the solution was stirred at room temperature for 17 hours. After the completion of the reaction, the reaction solution was poured into water and extracted three times with chloroform. The combined chloroform layer was washed with a saturated sodium chloride solution, dried over anhydrous sodium sulfate, filtered and concentrated. The resulting crude product was recrystallized from methanol to obtain 1.28 g (corresponding to 3.67 mmol) of 6-bromo-2-[4 -(3-hydroxypropoxy)phenyl]imidazo[1,2-a]pyridine (Fig. 11, Step 3).

100 mg (corresponding to 0.288 mmol) of 6-bromo-2-[4-(3-hydroxypropoxy)phenyl]imidazo[1,2-a]pyridine was dissolved in 4.0 mL of dioxane, and 2.0 mL of triethylamine was added thereto. Then, 0.22 mL (corresponding to 0.43 mmol) of bis(tributyltin) and 22.0 mg (a catalytic amount) of tetrakis-triphenylphosphine palladium were added thereto. After the reaction mixture was stirred at 90˚C for 24 hours, the solvent was distilled off under reduced pressure, and the residue was purified by flash silica gel column chromatography (elution solvent: hexane/ethyl acetate = 3/1) to obtain 68.0 mg (corresponding to 0.122 mmol) of 6-tributylstannyl-2-[4 -(3 - hyroxypropoxy)phenyl]imidazo[1,2-a]pyridiner (Fig. 11, Step 4).

The NMR measurement results of the resulting 6-tributylstannyl-2-[4 -(3 - hydroxypropoxy)phenyl]imidazo[1,2-a]pyridine (internal standard: tetramehtylsilane) are shown below.

NMR apparatus employed: JNM-ECP-500 (manufactured by Japan Electron Optics Laboratory Co., Ltd. (JEOL)) $^1$H-NMR (solvent: chloroform-dl; resonance frequency: 500 MHz): δ 7.97 (s, 1H), 7.88 (d, J = 8.3 Hz, 2H), 7.74 (s, 1H), 7.58 (d, J 8.3 Hz, 1H), 7.14 (d, J = 8.7 Hz, 1H), 6.98 (d, J = 8.7 Hz, 2H), 4.18 (t, J = 6.0 Hz, 2H), 3.89 (t, J = 6.0 Hz, 2H), 2.08 (tt, J = 6.0, 6.0 Hz, 2H), 1.59-1.49 (m, 6H), 1.39-1.31 (m, 6H), 1.18-1.05 (m, 6H), 0.90 (t, J = 7.3 Hz, 9H).

Example 19: Synthesis of 2-[4-(3-hydroxypropoxy)phenyl]-6-[$^{123}$I]iodoinidazo[1,2-a]pyridine

To 100 μL of a solution of 6-tributylstannyl-2-[4-(3 -hydroxypropoxy)pheny]imidazo[1,2-a]pyridine (concentration: 1 mg/mL) in a mixed solution of methanol/dimethylsulfoxide (in a ratio of 9/1), 80 μL of 2 mol/L hydrochloric acid, 15 μL of 1 mmol/L sodium iodide, 120 μL of [$^{123}$I]sodium iodide of 414 MBq and 20 μL of 10 %, (w/v) hydrogen peroxide were added. After the mixed solution, was left to stand at 50˚C for 10 minutes, the solution was subjected to HPLC under the following conditions, to obtain a fraction of 2-[4 -(3 - hydroxypropoxy)phenyl]-6-[$^{123}$I]iodoimidazo[1,2-a]pyridine.

HPLC conditions:

Column: Phenomenex Luna C18 (trade name; manufactured by Phenomenex Co.; size: 4.6 x 150 mm)
Mobile phase: 0.1 % trifluoroacetic acid .in water/0.1 % trifluoroacetic acid in acetonitrile = 80/20 to 0/100 (17 minutes)
Flow rate: 1.0 mL/min.
Detector: Ultraviolet visible absorptiometer (Detection wavelength: 282 nm) and radioactivity counter (manufactured by raptest: type STEFFI)

10 ml of water was added to the fraction. The resulting solution was passed through a reversed phase column (trade name: Sep-Pak (registered trademark) Light C8 Cartridges manufactured by Maters: the packed amount of the packing agent: 145 mg) so that the column adsorbs and collects 2-[4-(3 -hydroxypropoxy)phenyl]-6-[$^{123}$I]iodoimidazo [1,2-a]pyridine.The column was rinsed with 1 mL of water, and then 1 mL of diethyl ether was passed therethrough to elute 2-[4-(3-hydroxypropoxy)phenyl]-6-[$^{123}$I]iodoimidazo[1,2-a]pyridine. The amount of radioactivity of the obtained compound was 219 MBq at the end of synthesis. Further, the TLC analysis was conducted under the following conditions, and as a result, the radiochemical purity of the compound was 97%.

TLC analysis conditions:

TLC plate: Silica Gel 60 $F_{254}$ (trade name; manufactured by Merck & Co., Inc.)
Mobile phase: Chloroform/methanol/triethylamine = 100/1/2 Detector: Rita Star (trade name; manufactured by raytest)

Example 20 to 21, Comparative Example 7: Measurement of partition coefficient based on the octanol extraction method

A diethyl ether solution (Example 20) of Compound 4 prepared in Example 16, a diethyl ether solution (Example 21) of Compound 6 prepared in Example 19, and a diethyl ether solution (Comparative Example 7) of [$^{123}$I]-IMPY were each diluted with a 10 mg/mL ascorbic acid-containing physiological saline solution to adjust to a radioactive concentration of 20-30 MBq/mL. To 2 mL of octanol, 10 μL each of the prepared sample solution was added, and 2 mL of 10 mmol/L phosphate buffer (pH 7.4) was further added, followed by stirring for 30 seconds. After the mixture was centrifuged with

a low-speed centrifuge (2000 rpm x 60 min.), the octanol layer and the water layer were sampled each in an amount of 1 mL, and subjected to measurement of radioactivity count with an autowell gamma system (Type: ARC-301B, manufactured by Aloka). Using the obtained radioactivity count, $\log P_{octanol}$ was calculated in accordance with the equation (4).

$$\log P_{octanol} = \log_{10}\left(\frac{\text{Radioactivity count of octanol layer}}{\text{Radioactivity count of water layer}}\right) \cdots (4)$$

The results are shown in Table 6. A11 the compounds showed $\log P_{octanol}$ values between 1 and 3. It is known that compounds permeable to BBB show a $\log P_{octanol}$ value between 1 and 3 (Douglas D. Dischino et al., J, Nucl. Med., (1983), 24, p.1030-1038). From the above results, it is implied that both compounds have a BBB permeability comparable to IMPY.

Table 6: $\log P_{octanol}$ value of the present compound

| Experiment | Compound | $\log P_{octanol}$ value |
|---|---|---|
| Comparative Example 7 | [$^{123}$I]-IMPY | 2.1 |
| Example 20 | Compound 4 | 2.5 |
| Example 21 | Compound 6 | 2.1 |

Example 22 to 23, Example 8: Measurement of transferability into brain and clearance

Using Compound 4 and Compound 6, a time course change of radioactive accumulation in brain of male Wistar rats (7-week old) was measured.

Compound 4 (Example 22), Compound 6 (Example 23) and a solution of [$^{123}$I]-IMPY (Comparative Example 8) prepared above in Reference Example 1 were each diluted with a 10 mg/mL ascorbic acid-containing physiological saline solution to prepare solutions (20-31 MBq/mL in radioactive concentration). 0.05 mL each of the prepared sample solutions was injected under thiopental anesthesia into the tail vein of the respective Wistar rat (7-week old). The rats were sacrificed by bleeding from abdominal artery, and brains were removed and subjected to measurement of mass of brains and further subjected to measurement of radioactivity (hereinafter referred to as A in this Example) with a single channel analyzer (detector type: SP-20 manufactured by OHYO KOKEN KOGYO Co., Ltd.) 2, 5, 30 and 60 minutes after the injection. Further, the radioactivity level of the rest of the whole body was measured in the same manner as above (hereinafter referred to as B in this Example) . Using these measurement results, radioactive distribution per unit weight of brain (%ID/g) at the respective time points were calculated in accordance with the following formula (5). Three animals were used for the experiment at the respective time points.

$$\%ID/g = \frac{A}{B \times brain\ weight} \times 100 \quad \cdots (5)$$

The results are shown in Table 7. As shown in Table 7, Compounds 4 and 6 showed a significant accumulation like [$^{123}$I]-IMPY at the time point of two minutes after the injection, and then showed a tendency to rapidly clear away in 60 minutes. These results suggest that Compounds 4 and 6 possess high transferability to brain and rapid clearance from brain like [$^{123}$I]-IMPY.

Table 7: Radioactive distribution in brain of the present compound after intravenous injection (rats)

| Compound | | Radioactive distribution per unit weight (%ID/g) | | | |
|---|---|---|---|---|---|
| | | After 2 min. | After 5 min. | After 30 min. | After 60 min. |
| Example 22 | Compound 4 | 0.56 | 0.28 | 0.04 | 0.01 |
| Example 23 | Compound 6 | 0.81 | 0.56 | 0.07 | 0.02 |
| Comparative Example 8 | $^{123}$I-IMPY | 1.19 | 0.97 | 0.23 | 0.09 |

Example 24 to 25: Confirmation of imaging of amyloid in brain

(1) $A\beta_{1-42}$ (manufactured by Wako) was dissolved in phosphate buffer (pH 7.4) and shaken at 37˚C for 72 hours, to obtain 1 mg/mL of a suspension of aggregated Aβ (hereinafter referred to as amyloid suspension in the Examples).

(2) 2.5 μL (corresponding to 25 μg) of the amyloid suspension was injected into an amygdaloid nucleus on one side of a male Wistar rat (7-week old). As a control, 2.5 μL of phosphate buffered physiological saline solution (pH 7.4) was injected into an amygdaloid nucleus on the other side of the rat. The rats were examined 1 day after the injection of the amyloid suspension and the phosphate buffered physiological saline solution (pH 7.4).

(3) A sample solution (30 MBq/mL in radioactivity concentration. Example 24) in which Compound 4 was dissolved in a 10 mg/mL ascorbic acid-containing physiological saline solution and a sample solution (30 MBq/mL in radioactivity concentration. Example 25) in which Compound 6 was dissolved in a 10 mg/mL ascorbic acid-containing physiological saline solution were prepared. This solution was injected under thiopental anesthesia into the rat through the tail vein (dosage: 0.5 mL, dosed radioactivity: 11-15 MBq equivalent).

(4) Brain was removed 60 minutes after the injection to prepare a brain slice of 10 μm in thickness with a microtome (type: CM3050S, manufactured by LEICA). The brain slice was exposed to an imaging plate for 20 hours, and then image analysis was carried out by use of a Bio-imaging Analyser (type: BAS-2500; manufactured by FUJIFILM Corporation).

(5) After the completion of the image analysis using the Bio-imaging Analyzer, pathological staining with Thioflavin T was carried out to perform imaging by use of a fluorescence microscope (manufactured by NIKON Corporation; type: TE2000-U model; excitation wavelength: 400-440 nm; detection wavelength: 470 nm). Thus, it was confirmed that amyloid was deposited on the slice (Fig. 12 and Fig. 13).

Fig. 12 and Fig. 13 show images by autoradiogram and Thioflavin T staining of the brain slice of the rat to which amyloid was injected intracerebrally. As shown in these figures, a marked accumulation or radioactivity was observed in the amygdaloid nucleus on the side to which the amyloid suspension was injected, in both cases where Compounds 4 and 6 were administered. On the other hand, no significant accumulation of radioactivity was observed in the amygdaloid nucleus on the side to which the physiological saline solution was injected, compared with the other sites. On the auto-radiogram, little accumulation of radioactivity was observed at sites other than the site to which amyloid was injected. From the result of Thioflavin T staining, it was confirmed that amyloid was present in the site where radioactivity was accumulated (Fig. 12 and Fig. 13). These results suggest that Compounds 4 and 6 possess a property of accumulating on intracerebral, amyloid and a capability of imaging intracerebral amyloid.

Example 26 to 28: Reverse mutation test

In order to examine gene mutagenicity of Compounds 3, 5 and 8, reverse mutation test using *Salmonella typhimurium* TA98 and TA100 (hereinafter referred to as Ames test) was conducted.

The test was conducted without addition of S9mix and with addition of S9mix. Dimethylsulfoxide (hereinafter referred to as DMSO) was used as a negative control. A positive control was 2-(2-furyl)-3-(5-nitro-2-furyl)acrylamide (hereinafter referred to as AF-2) in case S9mix was not added, and 2-aminoanthracene (hereinafter referred to as 2-AA.) in case S9mix was added.

As a sample solution, to be added to a test plate, each compound was dissolved in BMSO to prepare a solution at a concentration of 50 mg/mL, and further, each solution was diluted with DMSO to prepare a solution with 7 dosage (geometric ratio 3). As a sample solution for preparing a sample of positive control, when A98 was used as a test strain without addition of S9mix, a solution of AF-2 (compound concentration: 1 μg/mL) in DMSO was prepared; when TA100 was used as a test strain without addition of S9mix, a solution of AF-2 (compound concentration: 0.1 μg/mL) in DMSO was prepared; when TA98 was used as a test strain with addition of S9mix, a solution of 2-AA (compound concentration: 5 μg/mL) in DMSO was prepared; and when TA100 was used as a test strain with addition of S9mix, a solution of 2-AA (compound concentration: 10 μg/mL) in DMSO was prepared.

After each sample solution to be examined and a test strain (TA98 or TA100) were mixed together so as to make the addition amount of each sample to be 0.1 mL/plate, the mixture was multilayered using soft agar on a medium of a test plate, and then incubated at 37°C for 48 hours. Separately, after each sample solution to be examined, S9mix and a test strain were mixed together so as to make the addition amount of each sample to be 0.1 mL/plate, the mixture was multilayered using soft agar on a medium of a test plate, and then incubated at 37°C for 48 hours. On the other hand, only DMSO was used as a sample solution to perform the same procedure as above for the negative control. Judgment was made by counting the number of reverse mutation colonies ion the plate after the incubation, and when the number of reverse mutation colonies was not less than two times the number in negative control and showed concentration-dependent increase, mutagenicity was determined to be positive.

Table 8: Results of Ames test

| | Compound | Mutagenicity | | | |
|---|---|---|---|---|---|
| | | Without addition of S9mix | | With addition of S9mix | |
| | | TA98 | TA100 | TA98 | TA100 |
| Example 26 | Compound 3 | Negative | Negative | Negative | Negative |
| Example 27 | Compound 5 | Negative | Negative | Negative | Negative |
| Example 28 | Compound 8 | Negative | Negative | Negative | Negative |

(Example 29 to Example 30) Confirmation of binding to amyloid

In order to study a binding mechanism of a compound of the present invention, an experiment for inhibition of binding to Thioflavin T was conducted using amyloid derived from amylin as a precursor compound. Amylin is an amyloid accumulating in pancreas of type II diabetes.

Method

(1) Amylin (human) (manufactured by Wake) was dissolved in phosphate buffer (pH 7.4) and shaken at 37°C for 72 hours, to obtain a 1 mg/mL suspension of aggregated amylin (hereinafter referred to as amyloid suspension in this Example).

(2) According to the method described in a literature (Naiki, H., et al., Laboratory Investigation 74, p.374-383 (1996)), the amyloid suspension was subjected to qualitative experiment based on fluorescence spectrophotometric method using Thioflavin T (manufactured by Fluka) to confirm that the aggregated amylin obtained in (1) was amyloid

(measurement conditions: excitation wavelength of 446 nm, and fluorescence wavelength of 490 nm).

(3) The amyloid suspension was dissolved in 50mM phosphate buffer (pH 7.4) at a concentration of amylin of 15 μM. Alto, Thioflavin T was dissolved at a concentration of 15 μM in 50 mM glycin-NaOH buffer (pH 8.5).

(4) Samples in which each compound for evaluation and amyloid were dissolved in a 50 mM phosphate buffer (pH 7.4) at final concentrations shown in Table 9 were prepared. 50 μL of each amyloid solution and Thioflavin T solution prepared in (3), and 50 μL of a sample solution Mere placed in each well (about 0..3 mL in volume) of a 96-well microplate.

Table 9: Final concentrations of each compound in sample solutions

| Experiment | Compound for evaluation | Concentration of compound for evaluation | Thioflavin T concentration | Amyloid |
|---|---|---|---|---|
| Example 29 | Compound 1 | Each concentration of 0, 1.5 15, 30 μmol/L | 5 μmol/L | 2.6 μmol/L |
| Example 30 | Compound 2 | | | |

(5) A sample in which 100 μL of 50 mM phosphate buffer (pH 7.4) and 50 μL of 50 mM glycin-NaOH buffer were mixed was made as a bank, and subjected to the same procedure as in (4) and used for a calculation of inhibition ratio.

(6) The microplate filled with the sample solutions was left to stand still at room temperature for 30 hours. Then, fluorescence strength of each sample solution was measured (measurement conditions: excitation wavelength of 446nm, emission wavelength of 490 nm) with microplate reader (type: SPECTRA MAX GEMINI XS, manufactured by Molecular Devices) (hereinafter, A denotes a fluorescence strength in a sample with zero (0) concentration of each compound for evaluation, and B denotes a fluorescence strength in a sample with 1.5 μmol/L or higher concentration of each compound for evaluation).

(7) Using the fluorescence strength measured above in (6), the following formula (6):

$$\text{Inhibition Ratio} = \frac{B-BG}{A-BG} \times 100 \quad (\%) \quad \cdots (6)$$

was used to determine the inhibition ratio.

Inhibition ratio of each compound for evaluation is shown in Table 10. Compounds 1 and 2 both inhibited the binding of Thioflavin T at any concentration. The results have shown that Compounds 1 and 2 competitively inhibit the binding of Thioflavin T. It is generally known that Thioflavin T binds to amyloid with recognition of its β-sheet structure. Thus, it has been suggested that Compounds 1 and 2 have the same mechanism of binding to amyloid as Thioflavin T, namely, binds thereto with recognition of the β-sheet structure.

Table 10: Inhibition ratio (%) of binding of amyloid (amylin) to Thioflavin T by the present compounds

| Experiment | Compound for evaluation | Inhibition ratio (%) | | |
|---|---|---|---|---|
| | | Concentration of compound for evaluation 1.5 μmol/L | Concentration of compound for evaluation 15 μmol/L | Concentration of compound for evaluation 30 μmol/L |
| Example 29 | Compound 1 | 34.4 | 51.3 | 46.8 |
| Example 30 | Compound 2 | 37.1 | 46.9 | 53.0 |

(Example 31) Measurement of radioactive distribution ratio in each organ

In order to confirm that a compound of the present invention can be distributed in a target organ and has good clearance to the outside of the body, Compound 1 was used to measure a time-course change of radioactive accumulation to each organ in SD rat (8 weeks).

A solution of Compound 1 prepared in Example 3 in diethylether was diluted with a 10 mg/mL ascorbic acid-containing physiological saline solution (Example 10) and adjusted to 8-12 MBq/mL in radioactive concentration. 0.05 mL each of the prepared sample solution was administered under thiopental anesthesia into the tail vein of the above rat. The rats were sacrificed by bleeding from abdominal artery, and each organ shown in Table 11 was removed 5, 30, 60 and 180 minutes after the administration. Each removed organ was subjected to measurement of mass and radioactivity with the same method as in Example 10. Also, radioactivity of the whole body of the rat after removal of the organs (hereinafter referred to as rest of the whole body) was measured. Using these measurement results, radioactive distribution per unit weight of each organ (%ID/g) at the respective time points was calculated in accordance with the following formula (7).

Three animals were used for the experiment at the respective time points.

$$\%ID/g = \frac{R^T}{(R^S + R^R) \times M^T} \times 100 \cdots (7)$$

$R^T$: Radioactivity of target organ (cpm)

$R^S$: Sum of radioactivity of all the organs (cpm)

$R^R$: Radioactivity of the rest of the whole body (cpm)

$M^T$: Mass of target organ (g)

The results are shown in Table 11. As shown in Table 11, Compound 1 was distributed to each organ at the time point of 5 minutes after administration, and then most of the radioactivity was distributed to small intestine and large intestine. In addition, radioactive distribution thereof was transferred from small intestine to large intestine. Thus, Compound 1 was found to be biliary-excreted rapidly after the administration and have a good clearance to the outside of the body.

Also, observing brain, heart, lung, pancreas and bone in which amyloid is considered to accumulate, obvious radioactive accumulation was found in all these organs at the time point of 5 minutes after the administration, and thus distribution of Compound 1 was confirmed. Moreover, the ratio of the time point of 5 minutes after the administration to the time point of 180 minutes after the administration ((%ID/g at the time point of 5 minutes after the administration)/ (%ID/g at the time point of 180 minutes after the administration)) showed high values such as 145 for brain, 20 for heart, 13 for lung, 24 for pancreas and 9 for bones. Thus, it has been shown that rapid radioactive distribution and rapid clearance are performed in organs in which amyloid is considered to accumulate.

From the above, it has been shown that a radioactive distribution at an early stage after administration and a rapid clearance to the outside of the body, which are required for an amyloid detecting reagent in a biological tissue, have been attained.

Table 11

| Tissue/ organ | 5 min. after administration | | 30 min. after administration | | 60 min. after administration | | 180 min. after administration | |
|---|---|---|---|---|---|---|---|---|
| | Average | Standard deviation | Average | Standard deviation | Average | Standard deviation | Average | Standard deviation |
| Blood | 0.420 | 0.028 | 0.129 | 0.010 | 0.101 | 0.008 | 0.068 | 0.009 |
| Brain | 0.755 | 0.051 | 01.049 | 0.002 | 0.014 | 0.002 | 0.005 | 0.001 |
| Heart | 0.582 | 0.043 | 0.081 | 0.009 | 0.043 | 0.006 | 0.029 | 0.004 |
| Lung | 0.661 | 0.047 | 0.117 | 0.011 | 0.076 | 0.009 | 0.053 | 0.010 |
| Liver | 1.865 | 0.091 | 0.293 | 0.042 | 0.113 | 0.008 | 0.051 | 0.005 |
| Spleen | 0.508 | 0.049 | 0.072 | 0.003 | 0.044 | 0.003 | 0.035 | 0.007 |

(continued)

| Tissue/ organ | 5 min. after administration | | 30 min. after administration | | 60 min. after administration | | 180 min. after administration | |
|---|---|---|---|---|---|---|---|---|
| | Average | Standard deviation | Average | Standard deviation | Average | Standard deviation | Average | Standard deviation |
| Pancreas | 0.778 | 0.029 | 0.097 | 0.013 | 0.051 | 0.008 | 0.033 | 0.006 |
| Stomach | 0.492 | 0.083 | 1.202 | 0.135 | 0.803 | 0.097 | 0.982 | 0.517 |
| Small intestine | 1.299 | 0.282 | 6.720 | 0.836 | 7.515 | 0.371 | 0.882 | 0.486 |
| Large intestine | 0.139 | 0.010 | 0.054 | 0.002 | 0.069 | 0.011 | 6.876 | 0.727 |
| Kidney | 1.212 | 0.092 | 0.432 | 0.068 | 0.236 | 0.034 | 0.070 | 0.002 |
| Adrenal gland | 2.471 | 0.346 | 0.199 | 0.007 | 0.068 | 0.012 | 0.052 | 0.005 |
| Bone | 0.282 | 0.013 | 0.066 | 0.005 | 0.047 | 0.004 | 0.032 | 0.006 |
| Marrow | 0.921 | 0.146 | 0.087 | 0.012 | 0.015 | 0.027 | 0.000 | 0.000 |
| Muscle | 0.372 | 0.025 | 0.042 | 0.003 | 0.025 | 0.004 | 0.015 | 0.003 |

Examples 32 to 33: Reverse mutation test

In order to examine gene mutagenicity of Compound 7 and 9, reverse mutation test using *Salmonella typhimurium* TA98 and TA100 (hereinafter referred to as Ames test) was conducted.

The test was conducted without addition of S9mix and with addition of S9mix. Dimethylsulfoxide (hereinafter referred to as DMSO) was used as a negative control. A positive control was 2-(2-furyl)-3-(5-nitro-2-furyl)acrylamide (hereinafter referred to as AF-2) in case S9mix was not added, and 2-aminoanthracene (hereinafter referred to as 2-AA) in case S9mix was added.

As a sample solution to be added to a test plate, each compound was dissolved in DMSO to prepare a solution at a concentration of 50 mg/mL, and further, each solution was diluted with DMSO to prepare a solution with 7 dosage (geometric ratio 3). As a sample solution for preparing a sample of positive control, when TA98 was used as a test strain without addition of S9mix, a solution of AF-2 (compound concentration: 1 μg/mL) in DMSO was prepared; when TA100 was used as a test strain without addition of S9mix, a solution of AF-2 (compound concentration: 0.1 μg/mL) in DMSO was prepared; when TA98 was used as a test strain with addition of S9mix, a solution of 2-AA (compound concentration: 5 μg/mL) in DMSO was prepared; and when TA100 was used as a test strain with addition of S9mix, a solution of 2-AA (compound concentration: 10 μg/mL) in DMSO was prepared.

After each sample solution to be examined and a test strain (TA98 or TA100) were mixed together so as to make the addition amount of each sample to be 0.1 mL/plate, the mixture was multilayered using soft agar on a medium of a test plate, and then incubated at 37°C for 48 hours. Separately, after each sample solution to be examined, S9mix and a test strain were mixed together so as to make the addition amount of each sample to be 0.1 mL/plate, the mixture was multilayered using soft agar on a medium of a test plate, and then incubated at 37°C for 48 hours. On the other hand, only DMSO was used as a sample solution to perform the same procedure as above for the negative control. Judgment was made by counting the number of reverse mutation colonies on the plate after the incubation, and when the number of reverse mutation colonies was not less than two times the number in negative control and showed concentration-dependent increase, mutagenicity was determined to be positive.

Table 12: Results of Ames test

| | Compound | Mutagenicity | | | |
|---|---|---|---|---|---|
| | | Without addition of S9mix | | With addition of S9mix | |
| | | TA98 | TA100 | TA98 | TA100 |
| Example 32 | Compound 7 | Negative | Negative | Negative | Negative |
| Example 33 | Compound 9 | Negative | Negative | Negative | Negative |

Examples 34-35: Micronucleus Test

In order to study mutagenicity (*in vivo*) of Compounds 3 and 7, induction of micronucleated polychromatic erythrocyte (hereinafter referred to as MNPCE) was examined using bone marrow cells of Crlj:CD1(ICR) male mouse.

0 mg/kg (negative control group), 250, 500, 1000 and 2000 mg/kg (test sample group) were set as doses for the test. Mice were sacrificed 24 and 48 hours after single oral administration, and bone marrow smear was prepared and observed. Further, a single dose of MMC at 2 mg/kg was abdominally administrated in positive control group, mice were sacrificed 24 hours after the administration, and then bone marrow smear was prepared and observed.

When appearance frequency of MNPCE in each administration group showed a dose-dependent increase or a statistically significant increase as compared to negative control group, positive determination, was given, and otherwise negative determination was given. As statistical analysis, significance tests were conducted by Wilcoxon rank sum test for the appearance frequency of MNPCE and the ratio of polychromatic erythrocyte (hereinafter referred to as PCE) to the total red blood cell (hereinafter referred to as RBC) in each administration group concerning the test sample and positive control groups relative to the negative control group and concerning these groups relative to each other where the significance level was set to less than 5% and less than 1%, respectively.

From the results of the test, no statistically significant difference was observed on the appearance frequency of MNPCE and the ratio of PCE to RBC for the test sample group as compared to the negative control group. On the other hand, a significant increase was observed on the appearance frequency of MNPCE for the positive control group as compared to the negative control group. Based on these results, mutagencity (*in vivo*) of the test sample was judged to be negative because no induction of micronucleus in mouse bone marrow cells was observed under the above test conditions using Compound 3 or 7.

(Example 36) Measurement of amyloid affinity

Affinity of the present compounds with amyloid was examined by the following *in vitro* binding tests.

Method

(1) Using methods described in Examples 29 and 30, a 1 mg/mL suspension of aggregated amylin was prepared (hereinafter referred to as amyloid suspension in this Example) was prepared.

Insulin with cross β-sheet structure was prepared by the following procedures (2) and (3) which was the method described in the known literature (Burke, M.J. et al., Biochemistry. 11, p. 2435-2439 (1972)) to which a little modification was made.

(2) 5 mg of insulin (manufactured by Stigma Aldrich Japan) was dissolved in 1 mL of deionized water which was adjusted to pH 2 with hydrochloric acid, and the solution was heated at 90 ˚C for 10 minutes, and then quickly cooled in dry ice/ethanol.

(3) A sample solution of (2) was heated at 90 ˚C for 5 minutes, and then quickly cooled in dry ice/ethanol. After repeating the same procedure for 10 times, change of a sample solution into gel form was visually confirmed.

(4) A sample of (3) was centrifuged (16000g X 15 min.), and then supernatant was removed whilst precipitates were dissolved with 1 mL of deionized water to obtain a suspension of aggregated insulin (hereinafter referred to as insulin amyloid suspension in this Example).

β2-microglobulin with cross β-sheet structure was prepared by the following procedures (5) and (6) which was the method described in the known literature (Ohhashi, Y. et al., Journal of Biological Chemistry. 280, p. 32843-32848 (2005)) to which a little modification was made.

(5) 50 mM glycin-HCl buffer (pH 2.5) containing 100mM NaCl was added to a 1 mg/mL solution of β2-microglobulin (manufactured by Oriental Yeast Co., ltd.), and then was subjected to ultrasonic treatment at 37˚C for 1 minute in an ultrasonic hot water tank (manufactured by Branson Ultrasonic, type: S1200 ) and an ultrasonic homogenizer (manufactured by SMT Co., Lid, type: UH-50), followed by heating at 37 ˚C for 9 minutes without ultrasonic treatment.

(6) After repeating the procedures of (5) for 17 times, the sample solution was subjected to the same treatment as above in (2). The sample solution was centrifuged (16000g X 15 min.), and then, supernatant was removed, and precipitates were dissolved in 0.5 mL of 50 mM glycin-HCl buffer (pH 2.5) containing 100 mM NaCl to obtain a suspension of aggregated β2 microglobulin (hereinafter referred to as β2m amyloid suspension in this Example).

(7) According to qualitative experiment based on fluorescence spectrophotometric method using Thioflavin T (manufactured by Fluka) described in Example 29 and Example 30, it was confirmed that the aggregated insulin and aggregated β2 microglobulin obtained in (4) and (6) were amyloid.

(8) A solution of Compound 1 which was synthesized by the method above in Example 3 was prepared (radioactivity of 500 MBq/mL), and diluted with a 0.1 % bovine serum albumin-containing phosphate buffer (pH 7.4) to prepare a solution of 1.2-112.9 pM in terms of the total amount of 2-[4-(2-hydroxyethoxy)phenyl]-6-iodoimidazo[1,2-a]pyridine.

(9) To each well of 96-well microplate, 50 μL of a solution prepared above in (8) (final concentration of 0.2-22.6 pM) and 50 μL of a solution in which a amylin amyloid suspension, insulin amyloid suspension or β2-m-amyloid suspension was diluted in a 0.1 % bovine serum albumin-containing phosphate buffer (pH 7.4) (final concentration of 0.8 μg/mL) were added, and then 150 μL of the same buffer was added.

(10) The microplate was shaken at a given rate (400 rpm) at 22 ˚C for 3 hours. Then, a mixture of each well was filtered through a glass fiber filter (trade name: Mulutiscreen™-FC, manufactured by Millipore), to separate the Compound 1 attached to amyloid from the free Compound 6.

(11) The glass fiber filter used for the filtration of the mixture was washed with a 0.1 % bovine serum albumin-containing phosphate buffer (0.2 mL x 5), and radioactivity of the glass fiber filter was measured with an autowell gamma system (manufactured by Aloka, Type: ARC-7001).

(12) From the measurement results of (11), relation between the amount of Compound 1 binding to amyloid and addition amount of amyloid was evaluated. For a non-specific binding, it was determined from a sample in which

no amyloid suspension was added above in (9)
(Example 36).

Relation of a concentration of Compound 1 in the sample solution and a radioactive count (CPM) on the glass fiber filter measured above in (11) was shown in Fig. 14. In the amyloid suspension added group (Fig. 14, a group with addition of amylin amyloid, a group with addition of insulin amyloid and a group with addition of β2-m-amyloid group), radioactivity was all high compared to the group without amyloid suspension (Fig. 14, amyloid non-added group), and a radioactivity of the glass fiber filter was increased proportionally to the addition concentration of Compound 1. In the conditions of this experiment, all amyloid (and amyloid to which Compound is attached) are larger than the pore size of the glass fiber. Thus, amyloid was supported on the glass fiber, and the radioactive count of the glass fiber became a value reflecting the amount of Compound 1 bound to amyloid. Since the value of the radioactive count of the glass fiber was increased with increase in concentration of Compound 1 and the radioactivity thereof was high compared to the group without addition of amyloid, it was shown that Compound 1 was a compound having a property of specifically binding to amyloid.

(Example 37) Measurement of binding to amyloid

The same procedures as in Example 36 was conducted except that a solution of Compound 6 was prepared (radioactivity of 500 MBq/mL), and diluted with a 0.1 % bovine serum albumin-containing phosphate buffer (pH 7.4) to prepare a solution of 0.5-59.7 pM in terms of the total amount of 2-[4-(2-hydroxypropoxy)phenyl]-6-iodoimidazo[1,2-a]pyridine as a sample solution.

Relation of a concentration of Compound 6 in the sample solution and a radioactive count (CPM) on the glass fiber filter was shown in Fig. 15. In the group with addition of amyloid suspension (Fig. 15, a group with addition of amylin amyloid, a group with addition of insulin amyloid and a group with addition of β2-m-amyloid group), a radioactivity was all high compared to the group without amyloid suspension (Fig. 15, amyloid non-added group), and a radioactivity of the glass fiber filter was increased proportionally to the addition concentration of Compound 6. In the conditions of this experiment, all amyloid (and amyloid to which Compound 6 is attached) are larger than the pore size of the glass fiber. Thus, amyloid was supported on the glass fiber, and the radioactive count of the glass fiber becomes a value reflecting the amount of Compound 6 bound to amyloid. Since the value of the radioactive count of the glass fiber was increased with increase in concentration of Compound 6 and the radioactivity thereof was high compared to the group without addition of amyloid, it was shown that Compound 6 was a compound having a property of specifically binding to amyloid.

## INDUSTRIAL APPLICABILITY

The reagent for detecting amyloid in biological tissues according to the present invention can be utilized as diagnostic agents for amyloid protein *in vitro* and *in vivo* in amyloidosis such as systemic amyloidosis.

## BRIEF DESCRIPTION OF THE DRAWINGS

Fig. 1 is a scheme of synthesis of 2-[4-(2-hydroxyethoxy)phenyl]-6-iodoimidazo[1,2-a]pyridine (non-radioactive iodinated form).
Fig. 2 is a scheme of synthesis of 6-tributylstannyl-2-[4-(2-hydroxyethoxy)phenyl]-imidazo[1,2-a]pyridine.
Fig. 3 is a scheme of synthesis of 2-(4-ethoxyphenyl)-6-iodoimidazo[1,2-a]pyridine (non-radioactive iodinated form).
Fig. 4 is a scheme of synthesis of 6-tributylstannyl-2-(4-ethoxyphenyl)imidazo[1,2-a]pyridine.
Fig. 5(a) is an autoradiogram of the brain slice after the injection of $^{123}$I-IMPY, and Fig. 5(b) is a fluorescent microscopic image of the Thioflavin T stained sample (a magnification of the site to which the amyloid suspension was injected).
Fig. 6(a) is an autoradiogram of the brain slice after the injection of 2-[4-(2-hydroxyethoxy)phenyl]-6-[$^{123}$I]iodoimidazo[1,2-a]pyridine, and Fig. 6(b) is a fluorescent microscopic image of the Thioflavin T stained sample (a magnification of the site to which the amyloid suspension was injected).
Fig. 7(a) is an autoradiogram of the brain slice after the injection of 2-(4-ethoxyphenyl)-6-[$^{123}$I]iodoimidazo[1,2-a]pyridine, and Fig. 7(b) is a fluorescent microscopic image of the Thioflavin T stained sample (a magnification of the site to which the amyloid suspension was injected).
Fig. 8 is a scheme of synthesis of 2-[3-(2-hydroxyethoxy)phenyl]-6-iodoimidazo[1,2-a]pyridine (non-radioactive iodinated form).
Fig. 9 is a scheme of synthesis of 6-tributylstannyl-2-[3-(2-hydroxyethoxy)phenyl]-imidazo[1,2-a]pyridine.

Fig. 10 is a scheme of synthesis of 2-[4 -(3 - hydroxypropoxy)phenyl]-6-iodoimidazo[1,2-a]pyridine (non-radioactive iodinated form).

Fig. 11 is a scheme of synthesis of 6-tributylstannyl-2-[4-(3-hydroxypropoxy)phenyl]-imidazo[1,2-a]pyridine.

Fig. 12(a) is an autoradiogram of the brain slice after the injection of Compound 4, and Fig. 12(b) is a fluorescent microscopic image of the Thioflavin T stained sample (a magnification of the site to which the amyloid suspension was injected).

Fig. 13(a) is an autoradiogram of the brain slice after the injection of Compound 6, and Fig. 13(b) is a fluorescent microscopic image of the Thioflavin T stained sample (a magnification of the site to which the amyloid suspension was injected).

Fig. 14 is a. graph which shows an ability of binding to amyloid of Compound 1.

Fig. 15 is a graph which shows an ability of binding to amyloid of Compound 6.

## Claims

**1.** A reagent for detecting amyloid deposited in a biological tissue, which comprises a compound represented by the following formula (1), or a salt thereof:

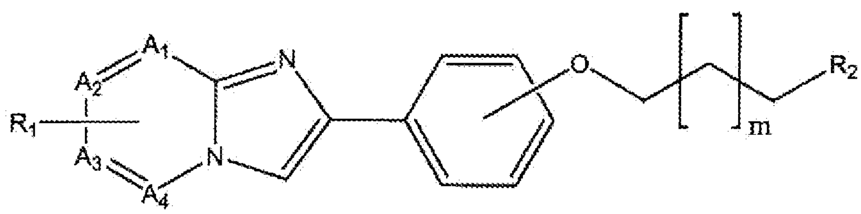

(1)

wherein $A_1$ $A_2$, $A_3$ and $A_4$ independently represent a carbon or nitrogen,
$R^3$ is a radioactive halogen substituent,
$R^2$ is a group selected from the group consisting or a hydrogen, hydroxyl group, methoxy group, carboxyl group, amino groups N-methylamino group, N,N-dimethylamino group and cyano group, and
In is an integer of 0 to 2,
provided that at least one of $A_1$, $A_2$, $A_3$ and $A_4$ represents a carbon, and $R^1$ binds to a carbon represented by $A_1$, $A_2$, $A_3$ or $A_4$.

**2.** The reagent according to claim 1, wherein at least three of $A_1$, $A_2$, $A_3$ and $A_4$ represent carbons.

**3.** The reagent according to claim 2, wherein, all of $A_1$, $A_2$, $A_3$ and $A_4$ represent carbons.

**4.** The reagent according to any one of claims 1 to 3, wherein $R^2$ is hydroxyl group.

**5.** The reagent according to any one of claims 1 to 4, wherein $R^1$ is selected from the group consisting of $^{18}F$, $^{75}Br$, $^{76}Br$, $^{123}I$, $^{124}I$, $^{125}I$, and $^{132}I$.

**6.** The reagent according to any one of claims 1 to 5, wherein the biological tissue is brain, heart, lung, pancreas, bone, or joint.

**7.** A process for production of a radioactive halogen-labeled organic compound, which comprises a step of preparing a reaction solution containing, together with a radioactive halogen ion, a compound represented by the following formula (2) or a salt thereof:

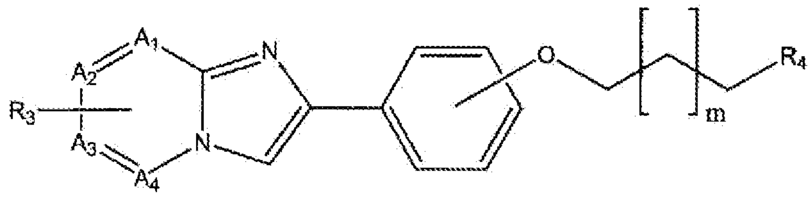

(2)

wherein $A_1$, $A_2$, $A_3$ and $A_4$ independently represent a carbon or nitrogen,
$R^3$ is a group selected from the group consisting of a non-radioactive halogen substituent, nitro substituent, trialkylammonium substituent having alkyl chains with 1 to 4 carbon atoms, trialkylstannyl substituent having alkyl chains with 1 to 4 carbon atoms and triphenylstannyl group,
$R^4$ is a group selected from the group consisting of a hydrogen, hydroxy group, methoxy group, carboxyl group, amino group, N-methylamino group, N,N-dimethylamino group and cyano group, and
In is an integer of 0 to 2,
provided that at least one of $A_1$, $A_2$, $A_3$ and $A_4$ represents a carbon, and $R^3$ binds to a carbon represented by $A_1$, $A_2$, $A_3$ or $A_4$, and
a step of giving a reaction condition to the reaction solution to synthesize a compound represented by the following formula (1) or a salt thereof:

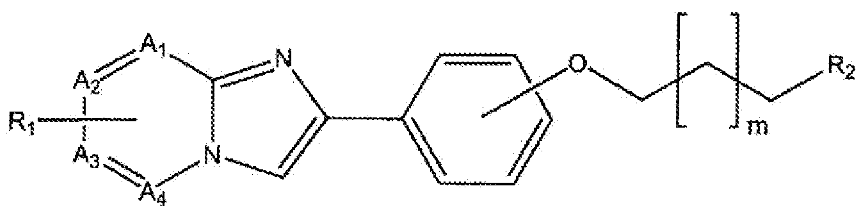

(1)

wherein $A_1$ $A_2$, $A_3$ and $A_4$ independently represent a carbon or nitrogen,
$R^1$ is a radioactive halogen substituent,
$R^2$ is a group selected from the group consisting of a hydrogen, hydroxyl group, methoxy group, carboxyl group, amino group, N-methylamino group, N,N-dimethylamino group and cyano group, and
m is an integer of 0 to 2,
provided that at least one of $A_1$, $A_2$, $A_3$ and $A_4$ represents a carbon, and $R^1$ binds to a carbon represented by $A_1$, $A_2$, $A_3$ or $A_4$.

**8.** The process for production of a radioactive halogen-labeled organic compound, according to claim 7, wherein at least three of $A_1$, $A_2$, $A_3$ and $A_4$ represent carbons.

**9.** The process for production of a radioactive halogen-labeled organic compound, according to claim 8, wherein all of $A_1$, $A_2$, $A_3$ and $A_4$ represent carbons.

**10.** The process for production of a radioactive halogen-labeled organic compound, according to any one of claims 7 to 9, wherein and $R^4$ are hydroxyl group.

**11.** The process for production of a radioactive halogen-labeled organic compound, according to any one of claims 7 to 10, wherein $R^3$ is selected from the group consisting of an iodine, bromine and trialkylstannyl substituent having alkyl chains with 1 to 4 carbon atoms and triphenylstannyl substituent, the radioactive halogen ion is selected from the group consisting of $^{1231}I$ ion, $^{124}I$ ion, $^{125}I$ ion and $^{123}I$ ion, and $R^1$ is selected from the group consisting of $^{123}I$, $^{124}I$, $^{125}I$ and $^{131}I$.

**12.** The process for production of a radioactive halogen-labeled organic compound, according to claim 11, wherein is selected from the group consisting of an iodine, trimethylstannyl substituent, tributylstannyl substituent and triphenylstannyl substituent.

**13.** The process for production of a radioactive halogen-labeled organic compound, according to any one of claims 7-10, wherein $R^3$ is a nitro substituent or trialkylammonium substituent having alkyl chains with 1 to 4 carbon atoms, the radioactive halogen ion is $^{16}F$ ion, and is $^{18}F$.

**14.** The process for production of a radioactive halogen-labeled organic compound, according to any one of claims 7-10, wherein $R^3$ is bromine, the radioactive halogen ion is $^{75}Br$ ion or $^{76}Br$ ion, and $R^1$ is $^{75}Br$ or $^{76}Br$.

**15.** A precursor compound for synthesizing a radioactive halogen-labeled organic compound, which is represented by the hollowing formula (2), or a salt thereof:

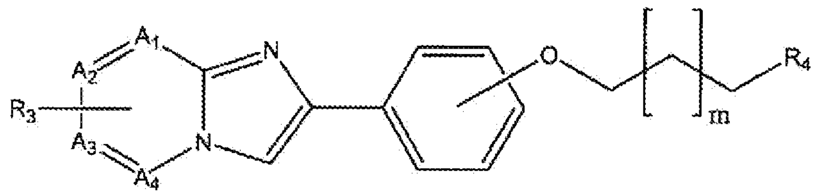

(2)

wherein $A_1$, $A_2$, $A_3$ and $A_4$ independently represent a carbon or nitrogen,
$R^3$ is a group selected from the group consisting of a non-radioactive halogen substituent, nitro substituent, trialkylammonium substituent having alkyl chains with 1 to 4 carbon atoms, trialkylstannyl substituent having alkyl chains with 1 to 4 carbon atoms and triphenylstannyl group,
$R^4$ is a group selected from the group consisting of a hydrogen, hydroxyl group, methoxy group, carboxyl group, amino group, N-methylamino group, N.N-dimethylamino group and cyano group, and
m is an integer on 0 to 2,
provided that at least one of $A_1$, $A_2$, $A_3$ and $A_4$ represents a carbon, and $R^3$ binds to a carbon represented by $A_1$, $A_2$, $A_3$ or $A_4$.

**16.** The precursor compound for synthesizing a radioactive halogen-labeled organic compound or a salt thereof, according to claim 15, wherein at least three of $A_1$, $A_2$ $A_3$ and $A_4$ represent carbons.

**17.** The precursor compound for synthesizing a radioactive halogen-labeled organic compound or a salt thereof, according to claim 16, wherein all of $A_1$, $A_2$ $A_3$ and $A_4$, represent carbons.

**18.** The precursor compound for synthesizing a radioactive halogen-labeled organic compound or a salt thereof, according to any one of claims 15-17, wherein $R^4$ is a hydroxyl group.

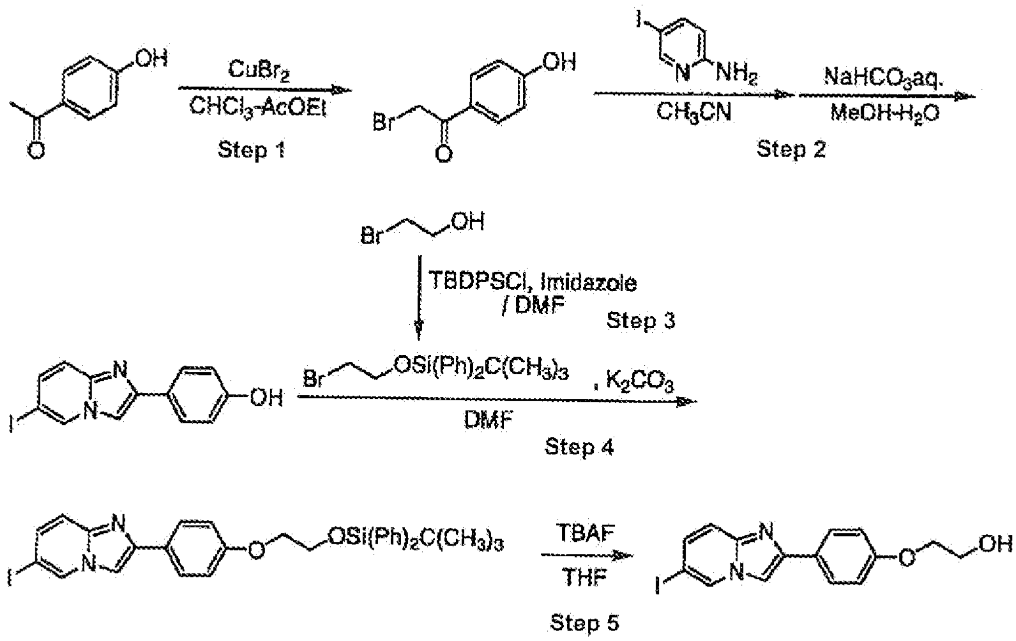


Figure 1

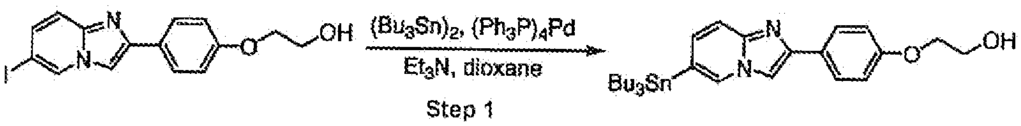


Figure 2

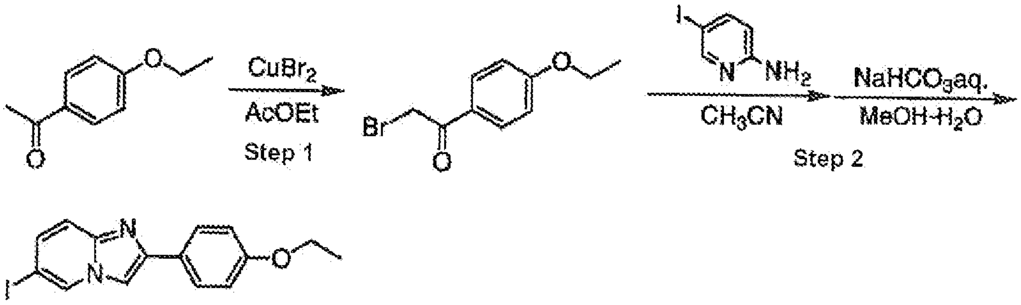


Figure 3

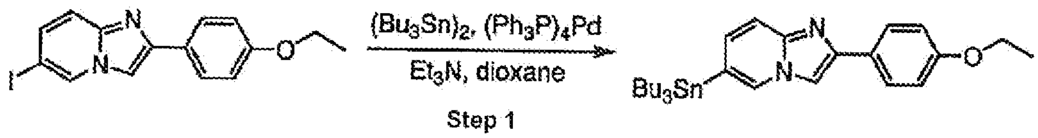


Figure 4

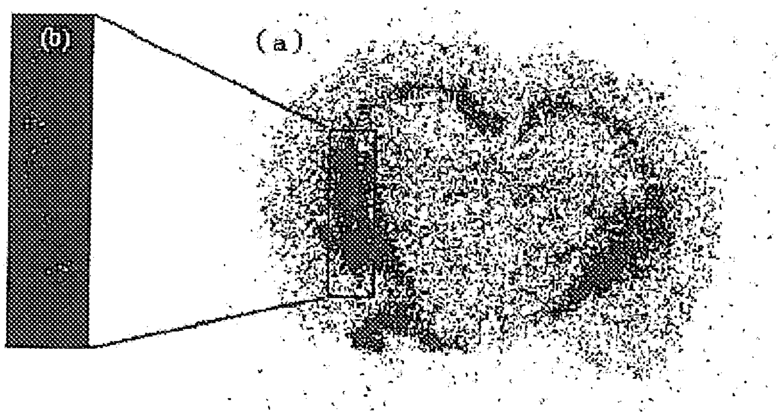


Figure 5

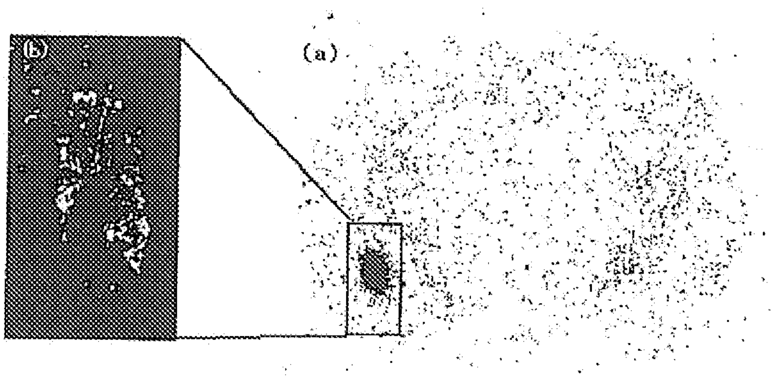


Figure 6

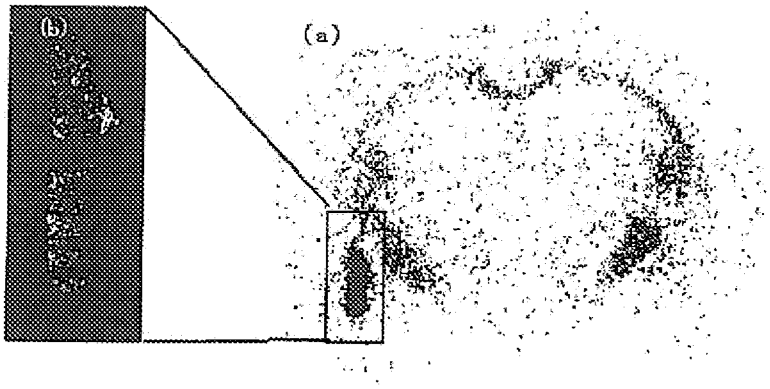


Figure 7

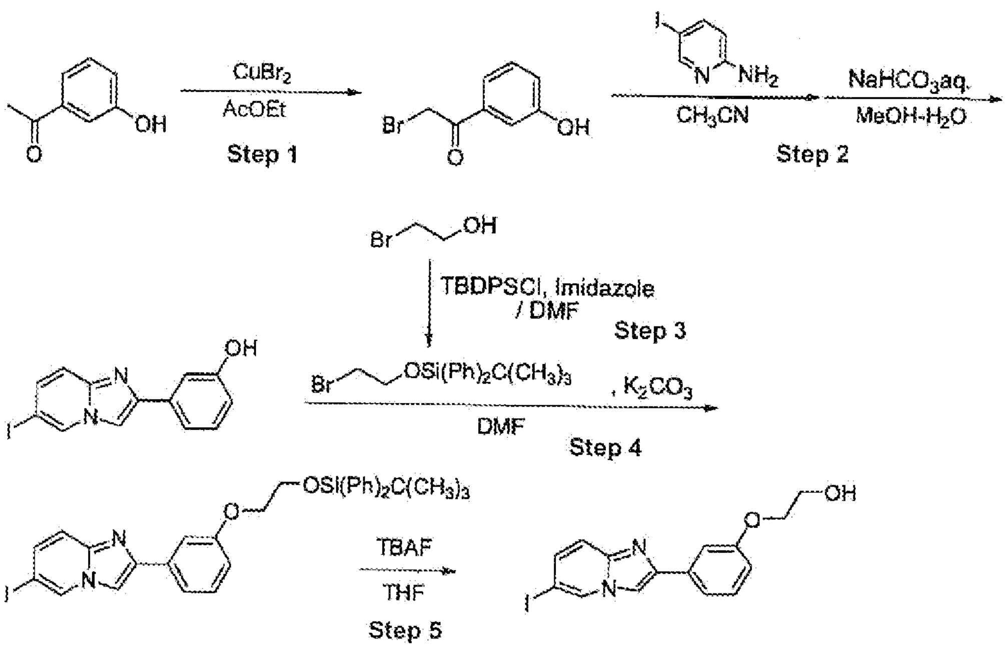


Figure 8

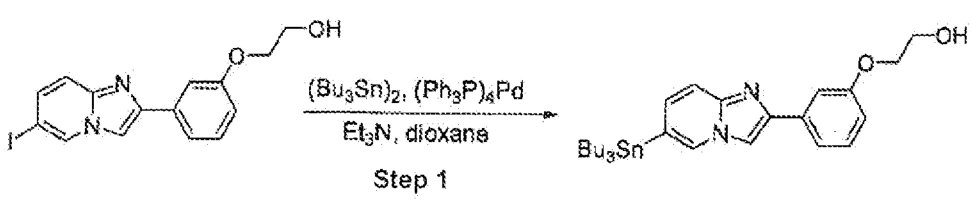


Figure 9

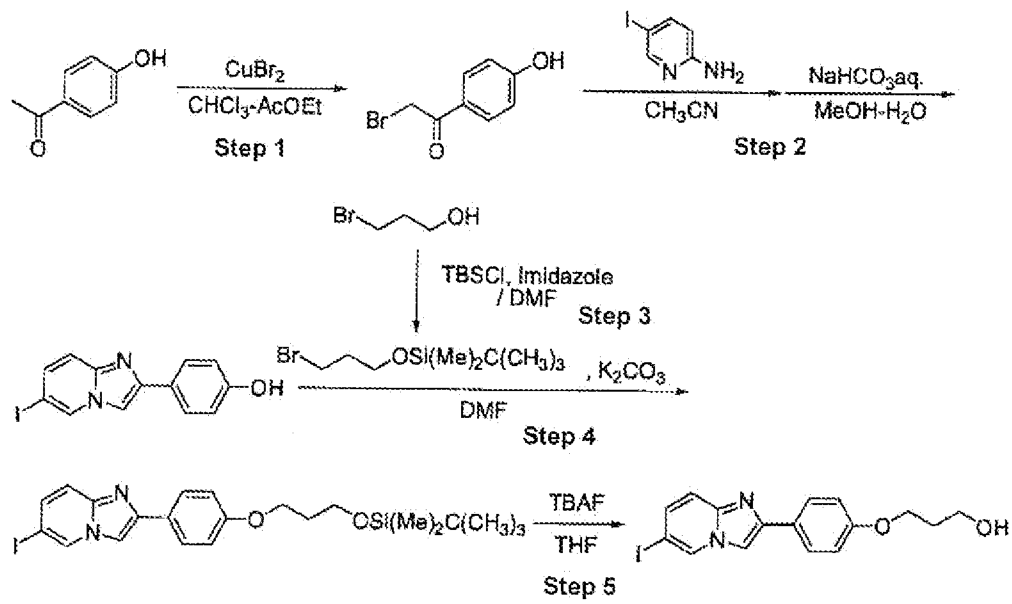


Figure 10

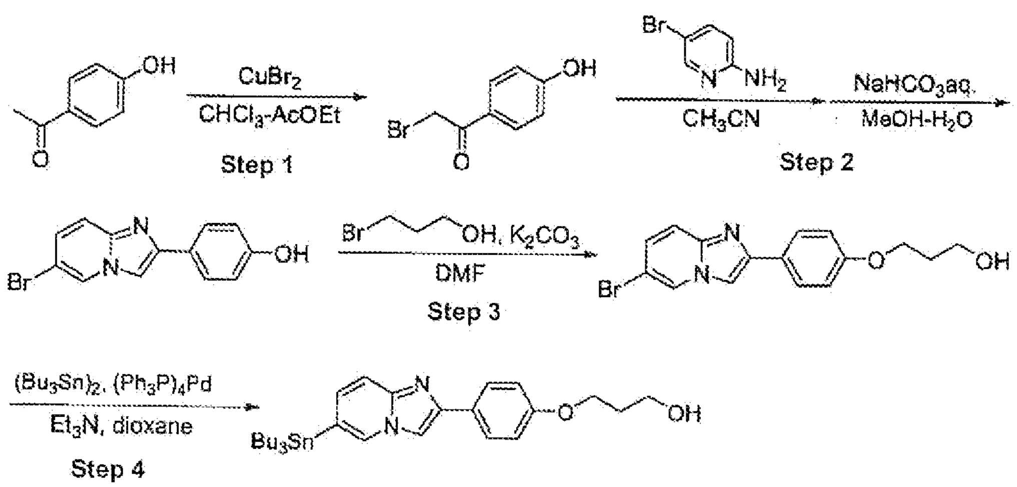


Figure 11

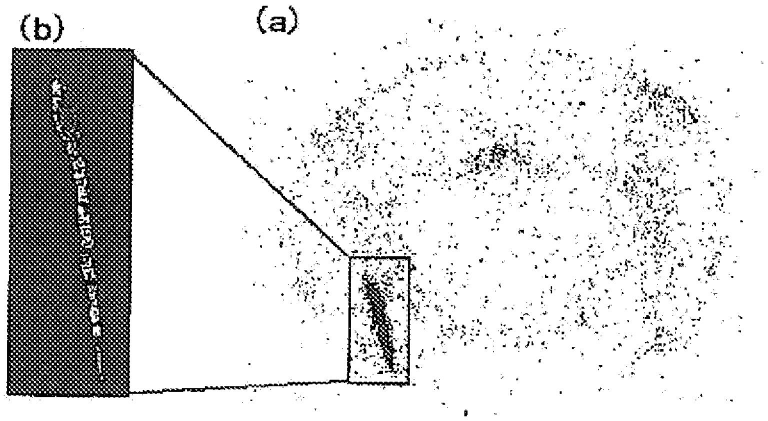


Figure 12

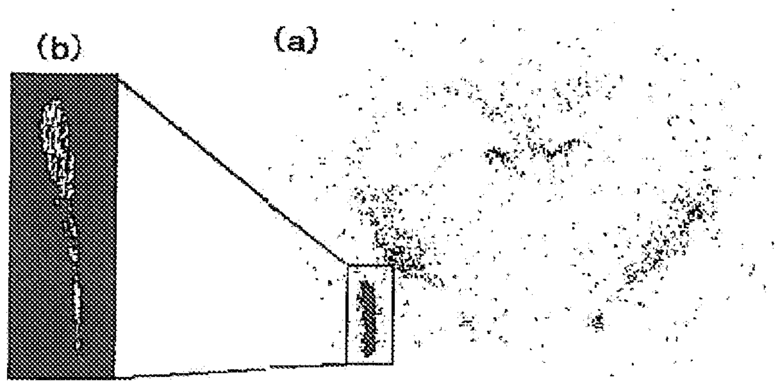


Figure 13

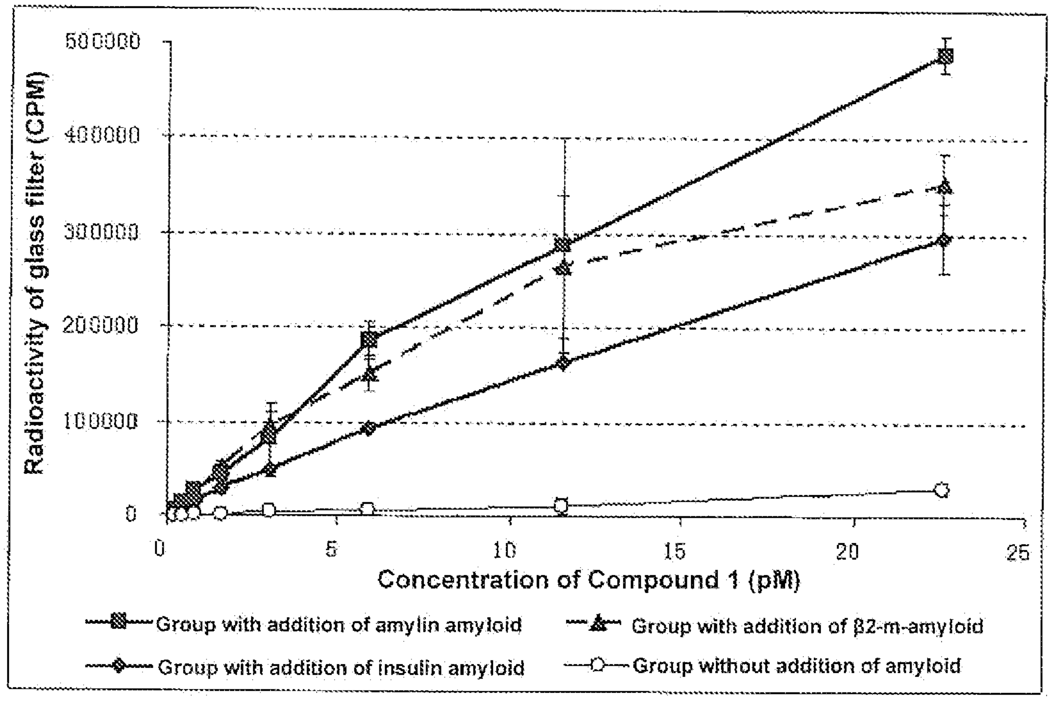


Figure 14

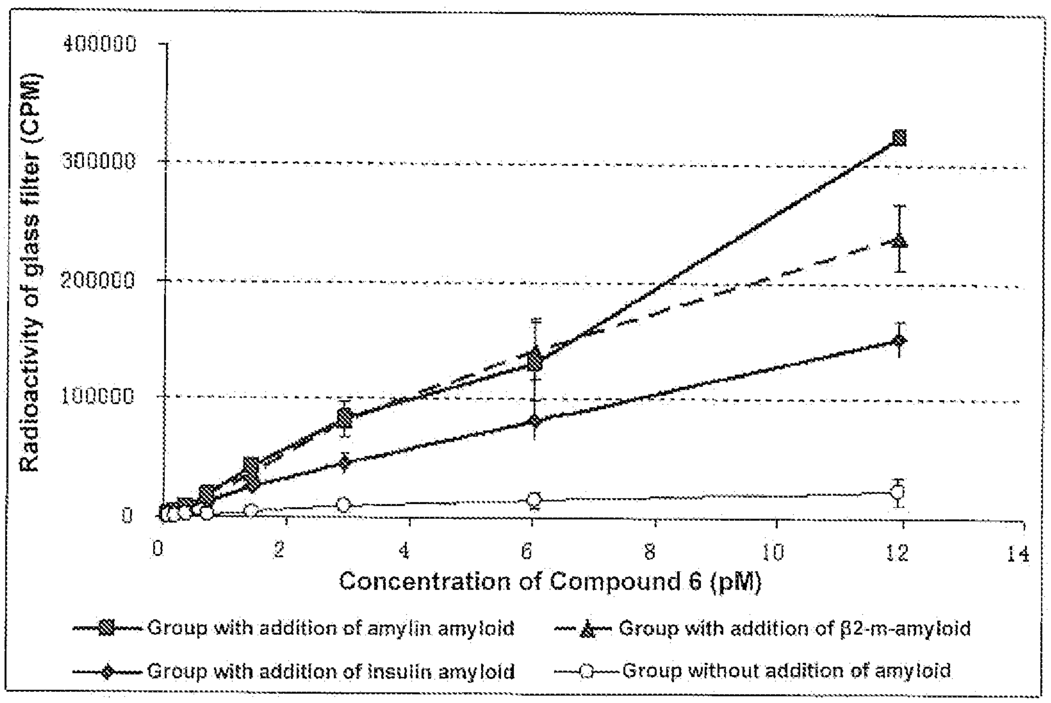


Figure 15

## INTERNATIONAL SEARCH REPORT

International application No.
PCT/JP2008/069504

A. CLASSIFICATION OF SUBJECT MATTER
*A61K51/00*(2006.01)i, *C07D471/04*(2006.01)i

According to International Patent Classification (IPC) or to both national classification and IPC

B. FIELDS SEARCHED

Minimum documentation searched (classification system followed by classification symbols)
A61K51/00, C07D471/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
BIOSIS(STN), CAplus(STN), EMBASE(STN), MEDLINE(STN), REGISTRY(STN), JSTPlus(JDreamII), JMEDPlus(JDreamII), JST7580(JDreamII)

C. DOCUMENTS CONSIDERED TO BE RELEVANT

| Category* | Citation of document, with indication, where appropriate, of the relevant passages | Relevant to claim No. |
|---|---|---|
| X<br>A | JP 63-91391 A (Ortho Pharmaceutical Corp.),<br>22 April, 1988 (22.04.88),<br>Claims; page 7, lower column; example 3<br>& US 4727145 A & US 4791117 A<br>& US 4833149 A & US 4871745 A<br>& EP 261912 A2 | 15-17<br>1-14,18 |
| X<br>A | WO 2001/074813 A2 (ORTHO MCNEIL PHARMACEUTICAL, INC.),<br>11 October, 2001 (11.10.01),<br>Claims<br>& US 2001/0051632 A1 & EP 1268478 A | 15-17<br>1-14,18 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12 December, 2008 (12.12.08) | 22 December, 2008 (22.12.08) |
| Name and mailing address of the ISA/<br>Japanese Patent Office<br>Facsimile No. | Authorized officer<br><br>Telephone No. |

Form PCT/ISA/210 (second sheet) (April 2007)

**INTERNATIONAL SEARCH REPORT**

International application No.
PCT/JP2008/069504

C (Continuation). DOCUMENTS CONSIDERED TO BE RELEVANT

| Category* | Citation of document, with indication, where appropriate, of the relevant passages | Relevant to claim No. |
|---|---|---|
| X<br>A | JP 2004-525192 A (F. Hoffmann-La Roche AG.),<br>19 August, 2004 (19.08.04),<br>Claims<br>& US 2003/0212096 A1 & EP 1381363 A<br>& WO 2002/092086 A1 | 15-17<br>1-14,18 |
| X<br>A | JP 2001-43978 A (Mitsui Chemicals, Inc.),<br>16 February, 2001 (16.02.01),<br>Claims; Par. No. [0012]<br>(Family: none) | 15,16<br>1-14,17,18 |
| X<br>A | WO 2005/066177 A1 (SCHERING PLOUGH LTD.),<br>21 July, 2005 (21.07.05),<br>Claims; compounds 87, 90, 93<br>& US 2005/0182059 A1 & EP 1699799 A<br>& WO 2005/066177 A1 | 15,16<br>1-14,17,18 |
| P,X | WO 2008/065785 A1 (Nihon Medi-Physics Co., Ltd.),<br>05 June, 2008 (05.06.08),<br>Full text<br>(Family: none) | 1-18 |
| P,X | WO 2007/135890 A1 (Nihon Medi-Physics Co., Ltd.),<br>29 November, 2007 (29.11.07),<br>Full text<br>(Family: none) | 1-18 |
| A | JP 2005-512945 A (The Trustees of the University of Pennsylvania),<br>12 May, 2005 (12.05.05),<br>& WO 2002/085903 A2 & EP 1381604 A2<br>& US 2004/0131545 A1 | 1-18 |
| A | JP 2005-526749 A (Novartis AG.),<br>08 September, 2005 (08.09.05),<br>& US 2005/0169837 A1 & EP 1483262 A<br>& WO 2003/074519 A1 | 1-18 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007002540 A
- WO 2007063946 A
- JP 2004506723 T
- JP 2005504055 T
- JP 2005512945 T
- JP 2002523383 T
- WO 2005016888 A
- WO 03106439 A


### Non-patent literature cited in the description


- **J. A. Hardy ; G. A. Higgins.** Alzheimer's Disease: The Amyloid Cascade Hypothesis. *Science,* 1992, vol. 256, 184-185
- **G. McKhann et al.** Clinical diagnosis of Alzheimer's disease: Report of the NINCDS-ADRDA Work Group under the auspices of Department of Health and Human Services Task Force on Alzheimer's Disease. *Neurology,* 1984, vol. 34, 939-944
- **Z.-P. Zhuang et al.** Radioiodinated Styrylbenzenes and Thioflavins as Probes for Amyloid Aggregates. *J. Med. Chem.,* 2001, vol. 44, 1905-1914
- **Masahiro Ono et al.** 11C-labeled stilbene derivatives as Aβ-aggregate-specific PET imaging agents for Alzheimer's disease. *Nuclear Medicine and Biology,* 2003, vol. 30, 565-571
- **H. F. Kung et al.** Novel Stilbenes as Probes for amyloid plaques. *J. American Chemical Society,* 2001, vol. 123, 12740-12741
- **Zhi-Ping Zhuang et al.** IBOX(2-(4-dimethylaminophenyl)-6-iodobensoxazole): a ligand for imaging amyloid plaques in the brain. *Nuclear Medicine and Biology,* 2001, vol. 28, 887-894
- **Furumoto Y et al.** [11C]BF-227: A New C-Labeled 2-Ethenylbenzoxazole Derivative for Amyloid-β Plaques Imaging. *European Journal of Nuclear Medicine and Molecular Imaging,* 2005, vol. 32 (1), 759
- **Eric D. Agdeppa et al.** 2-Dialkylamino-6-Acyimalononitrile Substituted Naphthalenes (DDNP Analogs) : Novel Diagnostic and Therapeutic Tools in Alzheimer's Disease. *Molecular Imaging and Biology,* 2003, vol. 5, 404-417
- **Zhi-Ping Zhuang et al.** Structure-Activity Relationship of Imidazo[1,2-a]pyridines as Ligands for Detecting β-Amyloid Plaques in the Brain. *J. Med. Chem,* 2003, vol. 46, 237-243
- **W. E. Klunk et al.** Imaging brain amyloid in Alzheimer's disease with Pittsburgh Compound-B. *Ann. Neurol.,* 2004, vol. 55, 306-319
- **Nicolaas P. L. ; G. Verhoeff et al.** In-Vivo Imaging of Alzheimer Disease β-Amyloid With [11C] SB-13 PET. *American Journal of Geriatric Psychiatry,* 2004, vol. 12, 584-595
- **Hiroyuki Arai et al.** [11C]-BF-227 AND PET to Visualize Amyloid in Alzheimer's Disease Patients. *Alzheimer's & Dementia: The Journal of the Alzheimer's Association,* 2006, vol. 2 (1), 312
- **Christopher M. Clark et al.** Imaging Amyloid with I123 IMPY SPECT. *Alzheimer's & Dementia: The Journal of the Alzheimer's Association,* 2006, vol. 2 (1), 342
- **D. M. Skovronsky et al.** *Proc. Natl. Acad. Sci.,* 2000, vol. 97, 7609
- **Zhi-Ping Zhuang et al.** *Nuclear Medicine and Biology,* 2001, vol. 28, 887-894
- **H. F. Kung et al.** *J. Am. Chem. Soc.,* 2001, vol. 123, 12740-12741
- **Masahiro One et al.** *Nuclear Medicine and Biology,* 2003, vol. 30, 565-571
- **King, L. Carroll ; Ostrum, G. Kenneth.** *Journal of Organic Chemistry,* 1964, vol. 29 (12), 3459-3461
- *Organic Syntheses,* 2004, vol. 10, 170
- *ORGANIC SYNTHESES,* 2002, vol. 79, 59
- *Organic Syntheses,* 1998, vol. 9, 417
- *ORGANIC SYNTHESES,* 1997, vol. 74, 248
- **Zhi-Ping Zhuang et al.** *J. Med. Chem,* 2003, vol. 46, 237-243
- **Naiki, H. et al.** *Laboratory Investigation,* 1996, vol. 74, 374-383
- **Wang, Y. et al.** *J. Labeled Compounds Radiopharmaceut.,* 2001, vol. 44, 239
- **Zhuang, Z.P. et al.** *J. Med. Chem.,* 2003, vol. 46, 237
- **Douglas D. Dischino et al.** *J. Nucl. Med.,* 1983, vol. 24, 1030-1038
- **Douglas D. Dischino et al.** *J, Nucl. Med.,* 1983, vol. 24, 1030-1038
- **Burke, M.J. et al.** *Biochemistry,* 1972, vol. 11, 2435-2439

- **Ohhashi, Y. et al.** *Journal of Biological Chemistry,* 2005, vol. 280, 32843-32848